# EUROPEAN PATENT APPLICATION

(11) **EP 4 843 994 A1**
(43) Date of publication of application: **25.11.2026**
(21) Application number: 25742241.0
(22) Date of filing: 15.01.2025
(51) Int. Cl.: C07K 5/11, A61P 21/00, A61P 3/04, A61P 1/16, A61P 3/10, A23L 33/18, A61K 38/00

(54) **NOVEL PEPTIDE HAVING MUSCLE FORMATION-PROMOTING ACTIVITY, ANTI-OBESITY ACTIVITY AND ANTI-DIABETIC ACTIVITY AND USES THEREOF**

(30) Priority: 18.01.2024 KR 20240008399
(71) Applicant: Caregen Co., Ltd., Seoul 06188 (KR)
(72) Inventor: CHUNG, Yong Ji, Seoul 06188 (KR); KIM, Eun Mi, Seoul 06188 (KR); KIM, Seon Soo, Seoul 06188 (KR); JEONG, Ah Reum, Seoul 06188 (KR)
(74) Representative: Heinonen & Co
(86) International application number: PCT/KR2025/000916
(87) International publication number: WO 2025/155102

(57) **Abstract**

A peptide of the present invention has muscle formation-promoting activity in myoblasts. The peptide of the present invention has the activities of inhibiting fat accumulation and promoting lipolysis in hepatocytes and adipocytes. The peptide of the present invention inhibits the expression of insulin resistance factors and increases the expression of insulin sensitivity factors in hepatocytes and adipocytes. Therefore, the peptide of the present invention can be used as an active substance for the treatment, prevention or amelioration of muscle diseases, obesity, fatty liver, and diabetes.

## Description

### [Technical Field]

### [Cross-citation with related applications]

This application claims the benefit of priority based on Korean Patent Application No. 10-2024-0008399 filed on January 18, 2024, and the entire contents of which are incorporated herein as part of this specification.

### [Technical Field]

The present invention relates to a novel peptide having muscle formation-promoting activity, anti-obesity activity and anti-diabetic activity, and the use thereof.

### [Background Art]

The decrease in skeletal muscle mass associated with aging leads to a decline in muscle strength and various physical functions. In particular, frailty, skeletal muscle atrophy, and sarcopenia are common among the elderly and are chronic problems threatening healthy life expectancy in every country; as a result, prevention, treatment, and rehabilitation have become major concerns in healthcare today. While muscle mass and strength naturally decline with age, sarcopenia involves an excessive reduction in muscle mass and strength even after accounting for age and gender leading to a decline in physical function and increased health risks or mortality. Recently, diagnostic criteria for sarcopenia have been established, and with its registration in the International Classification of Diseases (ICD) code, it is being classified as a disease rather than simply a result of aging.

Obesity refers to a condition in which there is an excessive amount of adipose tissue in the body, resulting from the accumulation of excess energy as body fat when the energy consumed through food is not balanced with the expenditure of that energy. According to the World Health Organization (WHO), more than one billion adults worldwide are overweight, and at least 3 million of them are clinically obese, a number that is increasing significantly in the United States and Europe. Overweight and obesity raise blood pressure and cholesterol levels, which can cause various diseases such as heart disease, diabetes, and arthritis, and increase the incidence of various adult diseases. Furthermore, overweight and obesity are contributing factor to an increased incidence of various adult diseases, such as arteriosclerosis, hypertension, hyperlipidemia, or heart disease, not only in adults but also in children and adolescents.

Currently, the representative obesity treatment drugs approved by the U.S. FDA and widely prescribed include a group of drugs that act on the central nervous system to function as appetite suppressants, and orlistat (Xenical), which is an inhibitor of lipase, a digestive enzyme secreted by the pancreas. Regarding central nervous system acting drugs, approvals for many drugs such as sibutramine have been revoked due to cardiovascular and psychiatric side effects, and orlistat has limitations in that its efficacy varies depending on fat intake along with various side effects. Meanwhile, as an endocrine peptide targeting drug, liraglutide, which is a glucagon-like peptide-1 (GLP-1) receptor agonist, has been approved and is in use, but the risk of thyroid cancer is emerging.

Diabetes is a type of metabolic disease characterized by a lack of insulin secretion or failure to function normally, resulting in hyperglycemia, which is an increase in the concentration of glucose in the blood. It causes various symptoms and signs due to hyperglycemia and leads to the excretion of glucose in the urine. Recently, the incidence of diabetes has been increasing explosively due to the rise in obesity rates, particularly abdominal obesity. Diabetes can be broadly classified into Type 1 diabetes, which is insulin-dependent, and Type 2 diabetes, which is insulin-independent. Type 2 diabetes is characterized by hyperglycemia, insulin resistance, and relative insulin secretion impairment.

When food is consumed, glucose is absorbed from the food in the digestive tract, stimulating the secretion of insulin from the beta cells of the pancreas, and the secreted insulin promotes the absorption of glucose into the muscles. In addition, while insulin is partially involved in glucose absorption by the liver, it primarily inhibits glucose production in the liver. Insulin lowers blood glucose levels by inhibiting glucose production in the liver and promoting glucose absorption into peripheral tissues, including muscles. Insulin resistance refers to a condition in which the blood glucose response to insulin is lower than normal under a given insulin concentration. Insulin regulates blood glucose by promoting glucose uptake into muscles or inhibiting glucose production in the liver, and insulin resistance refers to a state in which these insulin actions are reduced even in the absence of an insulin deficiency. Insulin receptors on the cell membrane are involved in the process of glucose absorption into cells of peripheral tissues; insulin resistance occurs when the number of insulin receptors decreases or intracellular defects develop after receptor binding. Although insulin receptor defects are found in type 2 diabetes, it is known that intracellular defects after receptor binding, specifically, impaired phosphorylation/dephosphorylation regulated by insulin play a much larger role. Among these mechanisms, it is known that impaired PI3K (Phosphoinositide 3-kinase) signaling reduces the movement of the glucose transporter GLUT-4 (Glucose transporter type 4) to the cell membrane.

Currently, lifestyle modifications (dietary therapy, exercise therapy) and drug therapy are used to control blood glucose. However, strict management and implementation of dietary therapy and exercise therapy are difficult, and their effectiveness is limited. Therefore, most diabetes patients rely on medications such as insulin, insulin secretagogues, insulin sensitivity enhancers, and blood glucose-lowering agents to control blood glucose, along with lifestyle modifications.

### [Prior Art Documents]

### [Patent Documents]

Korean Patent No. 10-2064387
Korean Patent No. 10-2486996
Korean Patent No. 10-2507392
International Patent Application No. PCT-KR2021-011278

### [Disclosure]

### [Technical Problem]

The inventors have made research efforts to develop a small molecule that has excellent therapeutic activity for diseases such as sarcopenia, diabetes, and obesity, while simultaneously ensuring safety and high drug delivery efficiency. As a result, it was experimentally confirmed that the peptide developed by the inventors has activity that promotes the expression of factors involved in muscle formation and factors involved in the process of muscle protein synthesis in muscle cells. In addition, the present invention was completed by confirming that the peptide of the present invention, in addition to these activities, has the activity of inhibiting fat accumulation in adipocytes and hepatocytes, promoting lipolysis, inhibiting insulin resistance, and improving insulin sensitivity.

Accordingly, it is an object of the present invention to provide a novel peptide having muscle formation-promoting, anti-obesity, or anti-diabetic activities.

It is another object of the present invention to provide a composition for promoting muscle formation, anti-obesity, anti-fatty liver, or anti-diabetes, comprising a peptide having the activity described above as an active ingredient.

It is still another object of the present invention to provide a pharmaceutical composition for the prevention or treatment of muscle diseases comprising a peptide having the activity described above as an active ingredient.

It is still another object of the present invention to provide a food composition for the prevention or amelioration of muscle diseases comprising a peptide having the activity described above as an active ingredient.

It is still another object of the present invention to provide a pharmaceutical composition for the prevention or treatment of obesity or fatty liver comprising a peptide having the activity described above as an active ingredient.

It is still another object of the present invention to provide a food composition for the prevention or amelioration of obesity or fatty liver comprising a peptide having the activity described above as an active ingredient.

It is still another object of the present invention to provide a pharmaceutical composition for the prevention or treatment of diabetes comprising a peptide having the activity described above as an active ingredient.

It is still another object of the present invention to provide a food composition for the prevention or amelioration of diabetes comprising a peptide having the activity described above as an active ingredient.

### [Technical Solution]

To achieve the objects of the present invention described above,
one aspect of the present invention provides a peptide comprising the amino acid sequence disclosed in SEQ ID NO: 1.

Another aspect of the present invention provides a composition for promoting muscle formation, anti-obesity, anti-fatty liver, or anti-diabetes, comprising the peptide as an active ingredient.

Another aspect of the present invention provides a pharmaceutical composition for the prevention or treatment of muscle diseases comprising the peptide as an active ingredient.

Still another aspect of the present invention provides a food composition for the prevention or amelioration of muscle diseases comprising the peptide as an active ingredient.

Still another aspect of the present invention provides a pharmaceutical composition for the prevention or treatment of obesity or fatty liver comprising the peptide as an active ingredient.

Still another aspect of the present invention provides a food composition for the prevention or amelioration of obesity or fatty liver comprising the peptide as an active ingredient.

Still another aspect of the present invention provides a pharmaceutical composition for the prevention or treatment of diabetes comprising the peptide as an active ingredient.

Still another aspect of the present invention provides a food composition for the prevention or amelioration of diabetes comprising the peptide as an active ingredient.

The present invention will be described in detail below.

### 1. Peptide and its activity

According to one aspect of the present invention, a peptide comprising the amino acid sequence disclosed in SEQ ID NO: 1 is provided.
[SEQ ID NO: 1]
HGTY(His-Gly-Thr-Tyr)

The term "peptide" as used herein refers to a linear molecule formed by amino acid residues being joined together by peptide bonds.

The peptide comprising the amino acid sequence of SEQ ID NO: 1 of the present invention may be used without modification, but variants or fragments of amino acids having different sequences may be used by deletion, insertion or substitution of amino acid residues, or a combination thereof, within a range that does not affect the original activity of the peptide, such as skin condition improvement activity.

The peptide of the present invention can be modified by phosphorylation, sulfation, acrylation, glycosylation, methylation, farnesylation, etc., within a range that does not change its activity.

The peptide of the present invention comprises a peptide comprising substantially identical amino acid sequence with the peptide comprising the amino acid sequence of SEQ ID NO: 1, and a variant thereof or an active fragment thereof. The substantially identical amino acid sequence means an amino acid sequence having at least 75%, for example, at least 80%, at least 85%, at least 90%, at least 95%, at least 97%, and at least 98% sequence identity with the amino acid sequence of SEQ ID NO: 1. In addition, the peptide may additionally include a targeting sequence, a tag, a labeled residue, or an amino acid sequence prepared for a specific purpose to increase half-life or peptide stability.

The peptide of the present invention may have N-terminal and/or C-terminal modifications induced to select a portion of the amino acid sequence and increase its activity. Through such N-terminal and/or C-terminal modifications, the stability of the peptide of the present invention can be significantly improved, for example, the half-life of the peptide can be increased upon in vivo administration. The term "stability" includes not only stability in vivo that protects the peptide of the present invention from attack by protein-cleaving enzymes in vivo, but also storage stability (e.g., room temperature storage stability).

The N-terminal modification may be a protecting group selected from the group consisting of an acetyl group, a fluorenylmethoxycarbonyl group, a formyl group, a palmitoyl group, a myristyl group, a stearyl group, and polyethylene glycol (PEG) attached to the N-terminus of the peptide. The C-terminal modification may be one in which a hydroxyl group (-OH), an amino group (-NH₂), a hydrazino group (-NHNH₂), etc. are attached to the C-terminus of the peptide, but is not limited thereto.

The peptide of the present invention can be prepared by various methods widely known in the field of the art to which this invention pertains. For example, the peptide of the present invention can be prepared according to a chemical synthesis method known in the industry, especially solid-phase synthesis techniques (Merrifield, J. Amer. Chem. Soc. 85:2149-54(1963); Stewart, et al., Solid Phase Peptide Synthesis, 2nd. ed., Pierce Chem. Co.: Rockford, 111(1984)) or liquid-phase synthesis techniques (US Patent No. 5,516,891).

The peptide of the present invention has activity that promotes muscle formation.

In one embodiment, the peptide of the present invention has the following activity with respect to activity that promotes muscle formation:
(i)activity that promotes the gene expression of cell proliferation-related factor PCNA (Proliferating cell nuclear antigen), NDRG2 (N-myc Downstream-Regulated Gene 2), or CDK4 (Cyclin-dependent kinase 4) in myoblasts;
(ii) activity that promotes the protein expression of cell proliferation-related factor SIRT1 (Sirtuin 1), Ki67, Pax7, or pAKT in myoblasts;
(iii) activity that promotes the gene expression of differentiation-related factor MyoD, Myf6, mTOR, Myf5, or Myf4 in myoblasts;
(iv) activity that promotes the protein expression of differentiation-related factor α-actinin, Myf6, or MyoG in myoblasts; or
(v) activity that promotes the protein expression of signaling factor involved in muscle protein synthesis, SIRT1, pAMPKα, pAKT, pmTOR, or p70S6K in myoblasts.

The peptide of the present invention has activity for inhibiting obesity or fatty liver.

In one embodiment, the peptide of the present invention has the following activity with respect to activity for inhibiting obesity or fatty liver:
(i) activity that inhibits lipid accumulation in hepatocytes;
(ii) activity that inhibits the protein expression of adipogenesis factor SREBP1 or FAS and promotes the protein expression of lipolysis factor pACC, pHSL, ATGL, or PLIN in hepatocytes where lipid accumulation is induced;
(iii) activity that promotes the gene expression of fatty acid oxidation-related factor CPT1, PGC1α, or PPARα and promotes the protein expression of fatty acid oxidation-related factor CPT1 or PGC1α in hepatocytes where lipid accumulation is induced;
(iv) activity that inhibits the gene expression of adipogenesis -related factor ACCα or PPAR-γ in adipocytes;
(v) activity that inhibits the protein expression of adipogenesis-related factor SREBP1, FAS, or C/EBPα and promotes the protein expression of fatty acid degradation-related factor pACCα in adipocytes;
(vi) activity that promotes the protein expression of lipolysis-related factor PLIN, pHSL, or PGC1α in adipocytes;
(vii) activity that promotes the gene expression of fatty acid oxidation-related factor PGC1α or CPT1 in adipocytes where insulin resistance is induced;
(viii) activity that inhibits the gene expression of fatty acid synthesis factor FAS and promotes the gene expression of lipolysis-related factor PLIN, HSL, or ATGL in adipocytes where insulin resistance is induced; or
(ix) activity that inhibits the protein expression of fatty acid synthesis factor FAS or SREBP1 and promotes the protein expression of lipolysis-related factor PLIN in adipocytes where insulin resistance is induced.

The peptide of the present invention has anti-diabetic activity.

In one embodiment, the peptide of the present invention has the following activity with respect to anti-diabetic activity:
(i) activity that promotes the protein expression of insulin sensitivity factor pIRS (Tyr612) or GLUT4 and inhibits the protein expression of insulin resistance factor pIRS (Ser302) or p70S6K in adipocytes;
(ii) activity that inhibits the gene expression of the insulin resistance factor P70S6K and promotes the gene expression of the insulin sensitivity factor AMPKα, GLUT4, or SIRT1 in adipocytes where insulin resistance is induced;
(iii) activity that promotes the protein expression of the insulin sensitivity factor pAMPKα and inhibits the protein expression of the insulin resistance factor pIRS (Ser302) in adipocytes where insulin resistance is induced;
(iv) activity that inhibits the gene expression of the insulin resistance factor mTOR or P70S6K and promotes the gene expression of the insulin sensitivity factor AKT, SIRT1, or AMPKα in hepatocytes where insulin resistance is induced; or
(v) activity that inhibits the protein expression of insulin resistance factor pmTOR or pJNK and promotes the protein expression of insulin sensitivity factor pAMPKα, GLUT4 or SIRT1 in hepatocytes where insulin resistance is induced.

### 1. Composition for promoting muscle formation

According to another aspect of the present invention, a composition for promoting muscle formation is provided, comprising a peptide comprising the amino acid sequence of SEQ ID NO: 1 as an active ingredient.

### Pharmaceutical composition

According to another aspect of the present invention, a pharmaceutical composition for the prevention or treatment of muscle diseases is provided, comprising as an active ingredient a peptide comprising the amino acid sequence of SEQ ID NO: 1.

The peptide comprising the amino acid sequence of SEQ ID NO: 1 is the same as the peptide described in item "*Peptide and its activity*" above, so the description of activity related to the prevention or treatment of muscle diseases is taken from therein and is not duplicated.

The peptide of the present invention having the activity described above exhibits excellent efficacy in inhibiting muscle loss and increasing muscle mass, and can be used for the prevention or treatment of muscle diseases.

Meanwhile, despite their effective biological activity, conventional functional peptides have shown disadvantages such as not being effectively absorbed into target tissues or cells due to the size of the peptide itself, or being eliminated from the body in a short period of time due to their short half-life. On the other hand, the pharmaceutical composition for the prevention or treatment of muscle diseases of the present invention includes a peptide consisting of about 10 or fewer amino acids as an active ingredient, and accordingly, the cell penetration rate of the active ingredient is excellent, and, for example, when administered topically, an effective preventive or therapeutic effect for muscle diseases can be obtained.

Herein, the term "muscle disease" refers to a muscle disease, condition, or state associated with a decrease in the proliferation or differentiation of myoblasts, and may collectively refer to muscle diseases resulting from, for example, muscle dysfunction, muscle atrophy, or muscle degeneration. The muscle disease may be one or more selected from the group consisting of, for example, muscular atrophy, senile sarcopenia, muscular dystrophy, disuse atrophy, spinal muscular amyotrophy, myotonia, muscular hypotonia, muscle weakness, muscle endurance weakness, amyotrophic lateral sclerosis, spinobulbar muscular atrophy, myasthenia gravis, myasthenia, muscle degeneration, and cachexia, but is not limited thereto.

In one embodiment, the peptide of the present invention can promote the proliferation and differentiation of myoblasts to inhibit muscle loss and increase muscle mass. Specifically, the peptide of the present invention can promote the proliferation of myoblasts and significantly increase the expression of early differentiation markers, mid-differentiation markers, and late differentiation markers of myoblasts, thereby promoting differentiation into muscle cells or muscle fibers. In addition, the peptide of the present invention can significantly increase the expression of myogenic markers and muscle protein synthesis markers, and can also increase the myoblast's myovascular synthesis ability, and can improve muscle mass recovery/regeneration when myoblasts are damaged by exogenous factors.

Herein, the term "prevention" refers to any act of suppressing or delaying the onset of a disease through the administration of the above composition.

Herein, the term "treatment" means any form of treatment that provides effects to an individual suffering from a disease or at risk of developing a disease, including improvement of the individual's condition (e.g., one or more symptoms), delay of disease progression, delay of symptom onset, or slowing of symptom progression. Accordingly, "treatment" and "prevention" are not intended to mean the cure or complete elimination of symptoms.

Herein, the term "administration" means introducing a specific substance into an individual by any appropriate method, and the pharmaceutical composition of the present invention may be administered through any general route capable of reaching a target in vivo. For example, the route of administration of the pharmaceutical composition of the present invention is not particularly limited, but may be administered orally or parenterally; in the case of parenteral administration, it may be administered by intramuscular injection, intravenous injection, subcutaneous injection, intraperitoneal injection, local administration, transdermal administration, etc., but is not limited thereto.

The pharmaceutical composition of the present invention may comprise a therapeutically effective amount of the peptide described above and a pharmaceutically acceptable carrier.

The term "therapeutic effective amount" means an amount sufficient to achieve the activity or efficacy of the peptide, which is an active ingredient of the pharmaceutical composition of the present invention, and, for example, an amount sufficient to achieve the preventive or therapeutic efficacy of diabetes or obesity.

The pharmaceutically acceptable carriers are those commonly used in formulations and include, but are not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oil.

In addition to the above components, the pharmaceutical composition of the present invention may additionally include, but is not limited to, lubricants, wetting agents, sweeteners, flavoring agents, emulsifiers, suspending agents, preservatives, etc.

Suitable pharmaceutically acceptable carriers and formulations are described in detail in Remington: The Science and Practice of Pharmacy (19th ed., 1995, Williams & Wilkins).

The pharmaceutical composition of the present invention may be prepared in a unit dose form or contained in a multi-dose container by formulation using pharmaceutically acceptable carriers and/or excipients according to methods readily practiced by a person skilled in the art to which the invention pertains. The formulation may be in the form of a solution, suspension, or emulsion in an oil or aqueous medium, or in the form of an extract, powder, granule, tablet, or capsule, and may additionally include a dispersant or a stabilizer.

The pharmaceutical composition of the present invention may be administered by any suitable route for treating muscle diseases, for example, orally or parenterally, and in the case of parenteral administration, may be administered by intravenous infusion, subcutaneous infusion, intramuscular infusion, intraperitoneal infusion, local administration, transdermal administration, etc.

The pharmaceutical composition of the present invention may include a peptide of the present invention at a concentration of 0.01 µM to 1000 µM, and specifically, the peptide of the present invention may be contained at a concentration of 0.01 µM to 1000 µM; 0.05 µM to 800 µM, 0.05 µM to 700 µM, 0.05 µM to 600 µM, 0.05 µM to 500 µM, 0.05 µM to 300 µM, 0.05 µM to 200 µM; 0.1 µM to 800 µM, 0.1 µM to 700 µM, 0.1 µM to 600 µM, 0.1 µM to 500 µM, 0.1 µM to 300 µM, 0.1 µM to 200 µM; 1 µM to 800 µM, 1 µM to 700 µM, 1 µM to 600 µM, 1 µM to 500 µM, 1 µM to 300 µM, 1 µM to 200 µM; 5 µM to 800 µM, 5 µM to 700 µM, 5 µM to 600 µM, 5 µM to 500 µM, 5 µM to 300 µM, or 5 µM to 200 µM , but is not limited thereto.

### Food composition

According to another aspect of the present invention, a food composition for the prevention or amelioration of muscle diseases is provided, comprising as an active ingredient a peptide comprising the amino acid sequence of SEQ ID NO: 1.

In one embodiment, the peptide of the present invention can promote the proliferation and differentiation of myoblasts to inhibit muscle loss and increase muscle mass. Accordingly, the peptide of the present invention can be effectively utilized as an active ingredient in a food composition for the prevention or amelioration of muscle diseases, and the food composition of the present invention can be effectively utilized for the prevention or amelioration of muscle diseases.

In this specification, the term "amelioration" may mean any action that at least reduces parameters related to the alleviation or treatment of a condition, such as the degree of symptoms.

The activity of the peptide of the present invention related to the prevention or amelioration of muscle diseases in the food composition of the present invention is the same as that described in the pharmaceutical composition described above, so it is taken from therein and is not duplicated.

In the functional food composition of the present invention, the peptide, which is the active ingredient, may be included in an appropriate amount selected within a range of 0.000001% to 10% by weight, specifically within the range of 10% by weight or less of the total weight of the composition.

The food composition of the present invention may include a food-effective amount of the peptide and a food-acceptable carrier.

The functional food composition of the present invention includes not only peptides as active ingredients but also ingredients that are typically added during food manufacturing, and may include, for example, proteins, carbohydrates, fats, nutrients, seasonings, and flavoring agents. Examples of the carbohydrates described above include monosaccharides, e.g., glucose, fructose, etc.; disaccharides, e.g., maltose, sucrose, oligosaccharides, etc.; and polysaccharides, such as conventional sugars like dextrin, cyclodextrin, etc., and sugar alcohols such as xylitol, sorbitol, erythritol, etc. As flavoring agents, natural flavoring agents, taumatin, stevia extract (e.g., rebaudioside A, glycyrrhizin, etc.), and synthetic flavoring agents (saccharin, aspartame, etc.) may be used.

In addition to the above-mentioned ingredients, the food composition of the present invention may contain various nutritional supplements, vitamins, minerals (electrolytes), flavoring agents such as synthetic and natural flavoring agents, coloring agents and thickening agents (cheese, chocolate, etc.), pectic acid and its salts, alginic acid and its salts, organic acids, protective colloidal thickeners, pH adjusters, stabilizers, preservatives, glycerin, alcohol, carbonating agents used in carbonated beverages, etc. In addition, it may contain fruit pulp for the manufacture of natural fruit juices, fruit juice beverages, and vegetable beverages. For example, when the functional food composition of the present invention is manufactured as a drink, in addition to the peptide which is the active ingredient of the present invention, it may additionally include citric acid, liquid fructose, sugar, glucose, acetic acid, malic acid, fruit juice, Eucommia ulmoides extract, jujube extract, licorice extract, etc.

There are no special restrictions on the types of the food compositions. Examples of the foods or food compositions include meat, sausage, bread, chocolate, candies, snacks, confectionery, pizza, ramen, other noodles, chewing gum, dairy products including ice cream, various soups, beverages, tea drinks, alcoholic beverages and vitamin complexes, dairy products, fermented milk, etc., and may include all functional foods or foods in the conventional sense.

Meanwhile, the food composition may include a health functional food or dietary composition. Herein, the term "health functional food" refers to a food manufactured and processed in the form of tablets, capsules, powders, granules, liquids, pills, etc., using raw materials or ingredients that have functionality useful to the human body. Herein, "functionality" means obtaining effects useful for health purposes, such as regulating nutrients or physiological actions regarding the structure and function of the human body.

The health functional food or dietary food may be manufactured by methods commonly used in the field of the art, and may be manufactured by adding raw materials and ingredients that are commonly added in the field of the art during such manufacturing. In addition, the formulation of the health functional food may also be manufactured without restriction as long as it is a formulation recognized as a health functional food. The food composition can be manufactured in various forms of formulations. Unlike general pharmaceuticals, it has the advantage of being made from food as a raw material, thus avoiding side effects that may occur with long-term use of pharmaceuticals, and it is highly portable. Therefore, the health functional food according to one embodiment can be consumed as an adjuvant to enhance the therapeutic effect of muscle diseases.

According to another aspect of the present invention, a method for treating muscle diseases in a subject in need thereof is provided, the method comprising administering to the subject an effective amount of the peptide comprising the amino acid sequence of SEQ ID NO: 1 or a pharmaceutical composition comprising the peptide as an active ingredient to a subject requiring treatment of muscle diseases.

According to another aspect of the present invention, a use of a peptide comprising the amino acid sequence of SEQ ID NO: 1 for the treatment or prevention of muscle diseases is provided.

According to another aspect of the present invention, a use of a peptide comprising the amino acid sequence of SEQ ID NO: 1 for the preparation of a pharmaceutical composition for the treatment or prevention of muscle diseases is provided.

In one embodiment, in connection with a composition for promoting muscle formation, a pharmaceutical composition or food composition for the prevention, treatment, or amelioration of muscle diseases of the present invention, the peptide of the present invention has the following activities:
(i) activity that promotes the gene expression of cell proliferation-related factor PCNA (Proliferating cell nuclear antigen), NDRG2 (N-myc Downstream-Regulated Gene 2), or CDK4 (Cyclin-dependent kinase 4) in myoblasts; (ii) activity that promotes the protein expression of cell proliferation-related factor SIRT1 (Sirtuin 1), Ki67, Pax7, or pAKT in myoblasts; (iii) activity that promotes the gene expression of differentiation-related factor MyoD, Myf6, mTOR, Myf5, or Myf4 in myoblasts; (iv) activity that promotes the protein expression of differentiation-related factor α-actinin, Myf6, or MyoG in myoblasts; or (v) activity that promotes the protein expression of signaling factor involved in muscle protein synthesis, SIRT1, pAMPKα, pAKT, pmTOR, or p70S6K in myoblasts.

### 3.Composition for anti-obesity, anti-fatty liver, and anti-diabetes

According to another aspect of the present invention, a composition for anti-obesity, anti-fatty liver, or anti-diabetes is provided, comprising a peptide comprising the amino acid sequence of SEQ ID NO: 1 as an active ingredient.

### Pharmaceutical composition

According to another aspect of the present invention, a pharmaceutical composition for the prevention or treatment of obesity or fatty liver is provided, comprising a peptide comprising the amino acid sequence of SEQ ID NO: 1 as an active ingredient.

As described above, the peptide of the present invention has activity that inhibits fat accumulation in hepatocytes and adipocytes and promotes lipolysis, so it can be used as an excellent preventive or therapeutic agent for obesity or fatty liver.

The peptide comprising the amino acid sequence of SEQ ID NO: 1 and its activity related to the treatment and prevention of obesity or fatty liver are the same as the peptide described in item "*Peptide and its activity*" above, so the specific description is taken from therein and is not duplicated.

According to another aspect of the present invention, a pharmaceutical composition for the prevention or treatment of diabetes is provided, comprising a peptide comprising the amino acid sequence of SEQ ID NO: 1 as an active ingredient.

In the present invention, the diabetes may be Type 1 diabetes or Type 2 diabetes, and specifically, may be Type 2 diabetes.

As described above, the peptide of the present invention can be used as an excellent preventive or therapeutic agent for diabetes by inhibiting the expression of insulin resistance factors and promoting the expression of insulin sensitivity factors in hepatocytes and adipocytes.

The peptide comprising the amino acid sequence of SEQ ID NO: 1 and its activity related to the treatment and prevention of diabetes are the same as the peptide described in item "*Peptide and its activity*" above, so the specific description is taken from therein and is not duplicated.

The pharmaceutical composition of the present invention may comprise a therapeutically effective amount of the peptide described above and a pharmaceutically acceptable carrier.

The term "therapeutically effective amount" means an amount sufficient for the peptide, which is an active ingredient of the pharmaceutical composition of the present invention, to achieve its activity or efficacy, and, for example, an amount sufficient to achieve efficacy in the treatment or prevention of diabetes or obesity.

The pharmaceutically acceptable carriers are those commonly used in formulations and include, but are not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oil.

In addition to the above components, the pharmaceutical composition of the present invention may additionally include, but is not limited to, lubricants, wetting agents, sweeteners, flavoring agents, emulsifiers, suspending agents, preservatives, etc.

Suitable pharmaceutically acceptable carriers and formulations are described in detail in Remington: The Science and Practice of Pharmacy, (19th ed., 1995, Williams & Wilkins).

The pharmaceutical composition of the present invention may be administered by any suitable route for treating diabetes, fatty liver, or obesity, for example, orally or parenterally, and in the case of parenteral administration, may be administered by intravenous infusion, subcutaneous infusion, intramuscular infusion, intraperitoneal infusion, local administration, transdermal administration, etc.

The dosage of the pharmaceutical composition may be 0.0001 µg to 100 mg, 0.001 µg to 100 mg, 0.01 µg to 100 mg, 0.1 µg to 100 mg, or 1.0 µg to 1000 mg per day, but is not limited thereto, and may be prescribed in various ways depending on factors such as the method of formulation, mode of administration, age, body weight, sex, pathological condition of the patient, food, time of administration, route of administration, excretion rate and response responsiveness.

The pharmaceutical composition of the present invention may be prepared in a unit volume form or contained in a multi-dose container by formulation using pharmaceutically acceptable carriers and/or excipients according to a method that can be easily carried out by a person skilled in the art to which the invention pertains. In this case, the formulation may be in the form of a solution, suspension, or emulsion in an oil or aqueous medium, or in the form of an extract, powder, granule, tablet, or capsule, and may additionally include a dispersant or a stabilizer.

### Food composition

According to another aspect of the present invention, a food composition for the prevention or amelioration of obesity or fatty liver is provided, comprising a peptide comprising the amino acid sequence of SEQ ID NO: 1 as an active ingredient.

As described above, the peptide of the present invention has the activity of inhibiting fat accumulation in hepatocytes and adipocytes and promoting lipolysis, so it can be used as an active ingredient in a food composition for the prevention or amelioration of obesity or fatty liver.

The peptide comprising the amino acid sequence of SEQ ID NO: 1 and its activity related to the prevention or amelioration of obesity or fatty liver are the same as the peptide and its activity described in the item "*Peptide and its activity*" above, so the specific description is taken from therein and is not duplicated.

According to another aspect of the present invention, a food composition for the prevention or amelioration of diabetes is provided, comprising a peptide comprising the amino acid sequence of SEQ ID NO: 1 as an active ingredient.

In the present invention, the diabetes may be Type 1 diabetes or Type 2 diabetes, and specifically, may be Type 2 diabetes.

As described above, the peptide of the present invention can be used as an active ingredient in a food composition for the prevention or amelioration of diabetes by inhibiting the expression of insulin resistance factors and promoting the expression of insulin sensitivity factors in hepatocytes and adipocytes.

The food composition for the prevention or amelioration of diabetes according to the present invention may be a functional food composition for regulating blood glucose levels.

In the functional food composition of the present invention, the regulation of blood glucose levels may be the regulation of blood glucose levels in patients with diabetes or high-risk patients in the pre-diabetic stage.

In the functional food composition of the present invention, the regulation of blood glucose levels may be the lowering of blood glucose levels.

In the food composition of the present invention described above, the peptide may be included in an appropriate amount selected within the range of 0.0001% by weight to 10% by weight with respect to the total weight of the composition.

In one embodiment, the functional food composition of the present invention may comprise a food-effective amount of the peptide and a food-acceptable carrier.

The food composition of the present invention includes not only peptides as the active ingredients but also ingredients that are typically added during food manufacturing, and may include, for example, proteins, carbohydrates, fats, nutrients, seasonings, and flavoring agents. Examples of the carbohydrates described above include monosaccharides, e.g., glucose, fructose, etc.; disaccharides, e.g., maltose, sucrose, oligosaccharides, etc.; and polysaccharides, e.g., conventional sugars such as dextrin, cyclodextrin, etc., and sugar alcohols such as xylitol, sorbitol, erythritol, etc. As flavoring agents, natural flavoring agents, taumatin, stevia extract (e.g., rebaudioside A, glycyrrhizin, etc.), and synthetic flavoring agents (saccharin, aspartame, etc.) may be used. The ratio of the carbohydrates may generally be about 1 to 20 g, preferably about 5 to 12 g, per 100 g of the food composition of the present invention, but is not limited thereto.

In addition to the above-mentioned ingredients, the food composition of the present invention may contain various nutritional supplements, vitamins, minerals (electrolytes), flavoring agents such as synthetic and natural flavoring agents, coloring agents and thickening agents (cheese, chocolate, etc.), pectic acid and its salts, alginic acid and its salts, organic acids, protective colloidal thickeners, pH adjusters, stabilizers, preservatives, glycerin, alcohol, carbonating agents used in carbonated beverages, etc. In addition, it may contain fruit pulp for the production of natural fruit juices, fruit juice beverages, and vegetable beverages. For example, when the functional food composition of the present invention is manufactured as a drink, in addition to the peptide which is the active ingredient of the present invention, it may additionally include citric acid, liquid fructose, sugar, glucose, acetic acid, malic acid, fruit juice, Eucommia ulmoides extract, jujube extract, licorice extract, etc.

In another aspect of the present invention, a method for treating, preventing, or improving obesity or fatty liver in a subject in need thereof is provided, the method comprising administering to the subject an effective amount of a peptide comprising the amino acid sequence of SEQ ID NO: 1 described above.

In another aspect of the present invention, a peptide comprising the amino acid sequence of SEQ ID NO: 1 described above is provided for use in the treatment, prevention, or amelioration of obesity or fatty liver.

In another aspect of the present invention, a peptide comprising the amino acid sequence of SEQ ID NO: 1 described above is provided for use in the preparation of a composition for the treatment, prevention, or amelioration of obesity or fatty liver.

In another aspect of the present invention, a method for treating, preventing, or improving diabetes in a subject in need thereof is provided, the method comprising administering to the subject an effective amount of a peptide comprising the amino acid sequence of SEQ ID NO: 1 described above.

In another aspect of the present invention, a peptide comprising the amino acid sequence of SEQ ID NO: 1 described above is provided for use in the treatment, prevention, or amelioration of diabetes.

In another aspect of the present invention, a peptide comprising the amino acid sequence of SEQ ID NO: 1 described above is provided for use in the preparation of a composition for the treatment, prevention, or amelioration of diabetes.

In one embodiment, in connection with the composition for anti-obesity or anti-fatty liver of the present invention and the pharmaceutical or food composition for the prevention, treatment, or amelioration of obesity or fatty liver, the peptide of the present invention has the following activities: (i) activity that inhibits lipid accumulation in hepatocytes; (ii) activity that inhibits the protein expression of adipogenesis factor SREBP1 or FAS and promotes the protein expression of lipolysis factor pACC, pHSL, ATGL, or PLIN in hepatocytes where lipid accumulation is induced; (iii) activity that promotes the gene expression of fatty acid oxidation-related factor CPT1, PGC1α, or PPARα and promotes the protein expression of fatty acid oxidation-related factor CPT1 or PGC1α in hepatocytes where lipid accumulation is induced; (iv) activity that inhibits the gene expression of adipogenesis-related factor ACCα or PPAR-γ in adipocytes; (v) activity that inhibits the protein expression of adipogenesis-related factor SREBP1, FAS, or C/EBPα and promotes the protein expression of fatty acid degradation-related factor pACCα in adipocytes; (vi) activity that promotes the protein expression of lipolysis-related factor PLIN, pHSL, or PGC1α in adipocytes; (vii) activity that promotes the gene expression of fatty acid oxidation-related factor PGC1α or CPT1 in adipocytes where insulin resistance is induced; (viii) activity that inhibits the gene expression of fatty acid synthesis factor FAS and promotes the gene expression of lipolysis-related factor PLIN, HSL, or ATGL in adipocytes where insulin resistance is induced; or (ix) activity that inhibits the protein expression of fatty acid synthesis factor FAS or SREBP1 and promotes the protein expression of lipolysis-related factor PLIN in adipocytes where insulin resistance is induced.

In one embodiment, in connection with the composition for anti-diabetes and the pharmaceutical or food composition for the prevention, treatment, or amelioration of diabetes, the peptide of the present invention has the following activities: (i) activity that promotes the protein expression of insulin sensitivity factor pIRS (Tyr612) or GLUT4 and inhibits the protein expression of insulin resistance factor pIRS (Ser302) or p70S6K in adipocytes; (ii) activity that inhibits the gene expression of the insulin resistance factor P70S6K and promotes the gene expression of the insulin sensitivity factor AMPKα, GLUT4, or SIRT1 in adipocytes where insulin resistance is induced; (iii) activity that promotes the protein expression of the insulin sensitivity factor pAMPKα and inhibits the protein expression of the insulin resistance factor pIRS (Ser302) in adipocytes where insulin resistance is induced; (iv) activity that inhibits the gene expression of the insulin resistance factor mTOR or P70S6K and promotes the gene expression of the insulin sensitivity factor AKT, SIRT1, or AMPKα in hepatocytes where insulin resistance is induced; or (v) activity that inhibits the protein expression of insulin resistance factor pmTOR or pJNK and promotes the protein expression of insulin sensitivity factor pAMPKα, GLUT4 or SIRT1 in hepatocytes where insulin resistance is induced.

### [Advantageous Effects]

A peptide of the present invention has muscle formation-promoting activity in myoblasts. The peptide of the present invention has the activities of inhibiting fat accumulation and promoting lipolysis in hepatocytes and adipocytes. In addition, the peptide of the present invention inhibits the expression of insulin resistance factors and increases the expression of insulin sensitivity factors in hepatocytes and adipocytes. Therefore, the peptide of the present invention can be used as an active substance for the treatment, prevention or amelioration of muscle diseases, obesity, fatty liver, and diabetes.

However, the effects of the present invention are not limited to those mentioned above, and other effects not mentioned will be clearly understood by those skilled in the art from the following description.

### [Description of Drawings]

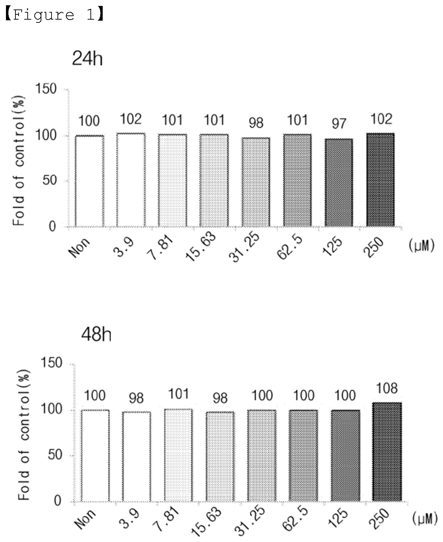

FIG. 1 is a diagram showing that the peptide of the present invention has cell proliferation-promoting activity in C2C12 cells.

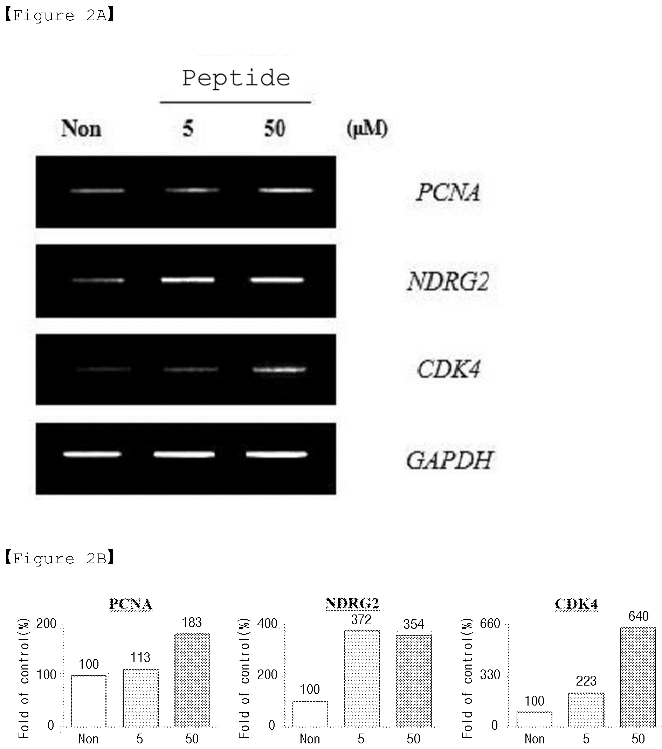

FIGS. 2A and 2B show the results of the peptide of the present invention promoting the expression of genes of cell proliferation-related factors PCNA (Proliferating cell nuclear antigen), NDRG2 (N-myc Downstream-Regulated Gene 2), and CDK4 (Cyclin-dependent kinase 4), in C2C12 cells. Non represents the peptide-untreated group, in which differentiation-induced C2C12 cells were not treated with the peptide.

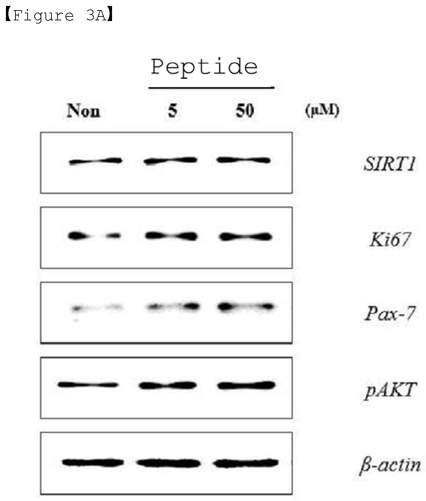

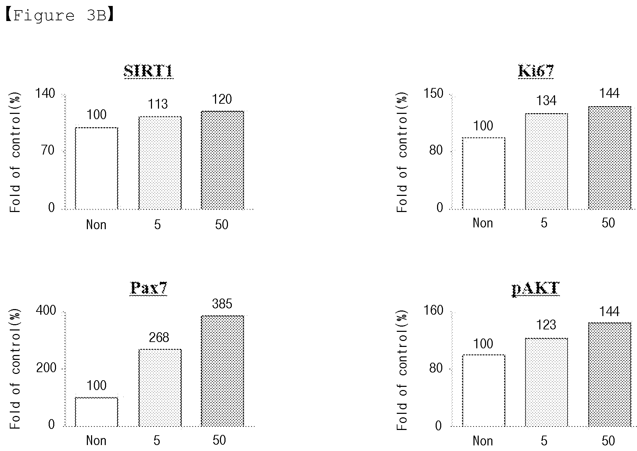

FIGS. 3A and 3B show the results of the peptide of the present invention increasing the expression levels of cell proliferation-related factors SIRT1 (Sirtuin1), Ki67, Pax7, and pAKT proteins in C2C12 cells. Non is the peptide-untreated group in which differentiation-induced C2C12 cells were not treated with the peptide.

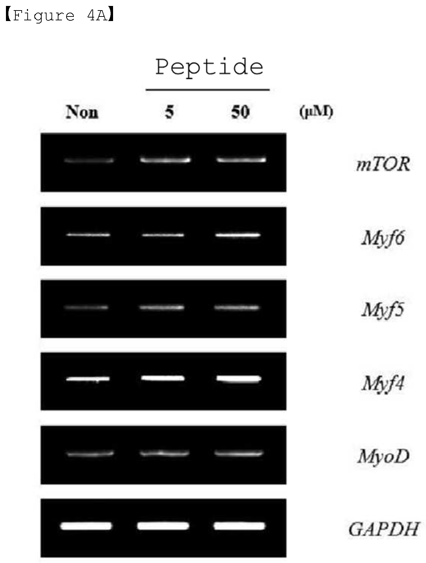

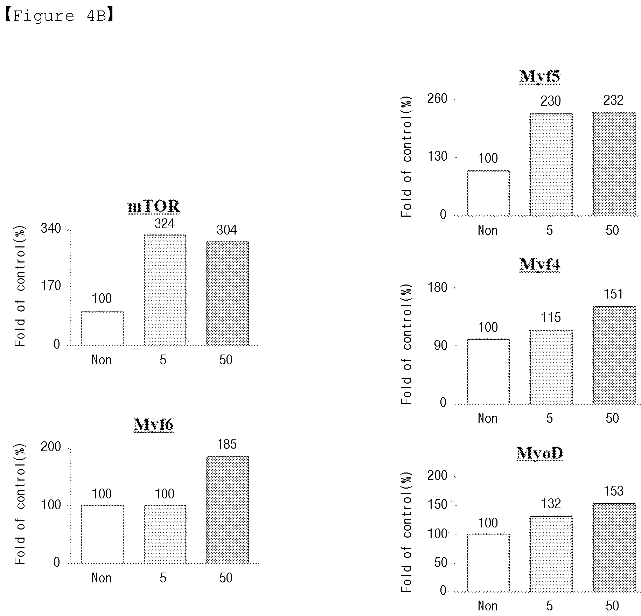

FIGS. 4A and 4B show the results of the peptide of the present invention increasing the expression levels of genes MyoD, Myf6, mTOR, Myf5, and Myf4, which are factors involved in mid- and late-differentiation in C2C12 cells. Non is the peptide-untreated group in which differentiation-induced C2C12 cells were not treated with the peptide.

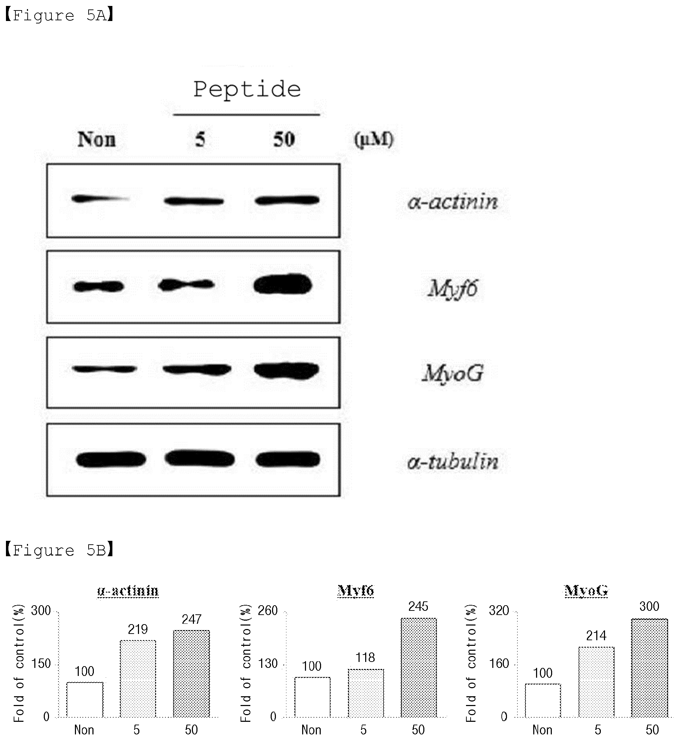

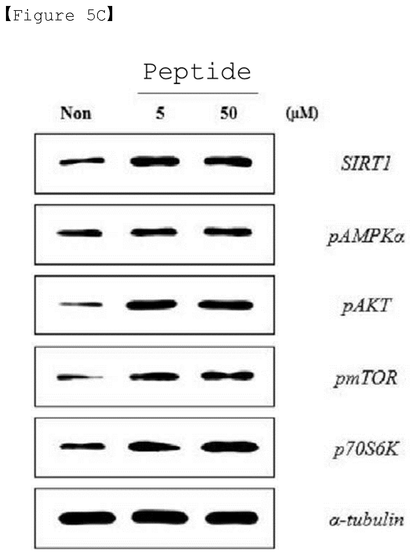

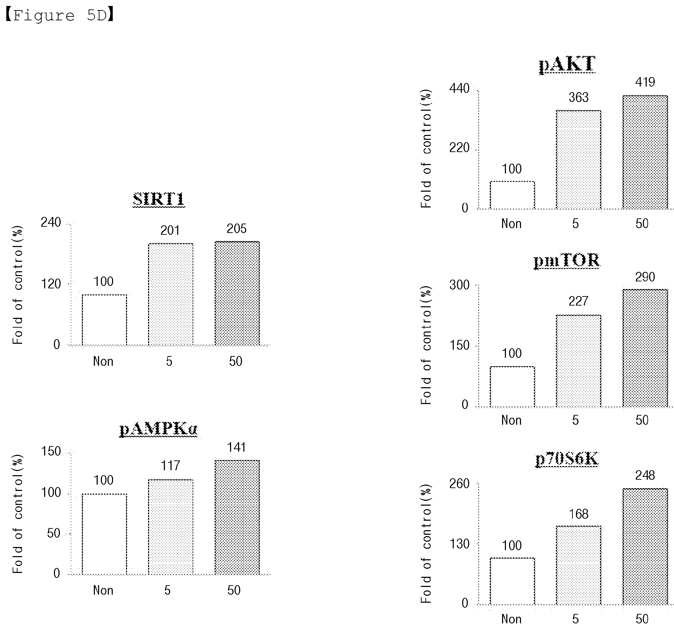

FIGS. 5A, 5B, 5C, and 5D show the results that the peptide of the present invention increases the expression of differentiation marker proteins α-actinin, Myf6, and MyoG in C2C12 cells, and increases the expression of signaling proteins involved in muscle protein synthesis, such as SIRT1, pAMPKα, pAKT, pmTOR, and p70S6K. Non is the peptide-untreated group in which differentiation-induced C2C12 cells were not treated with the peptide.

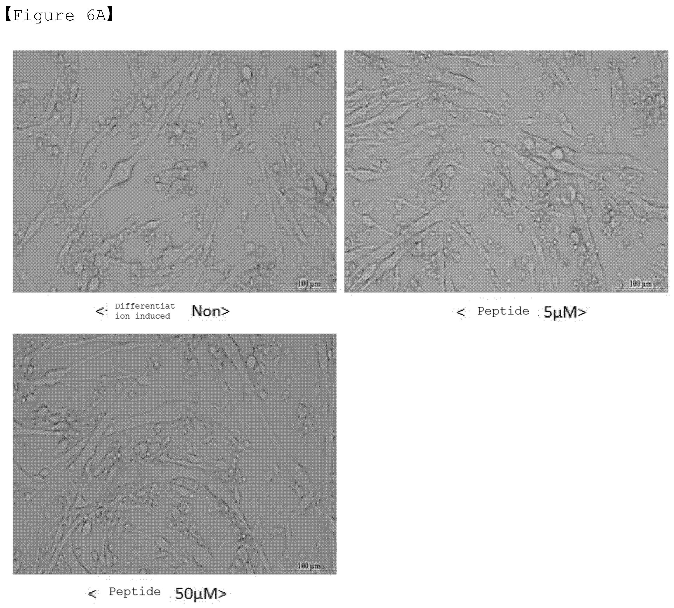

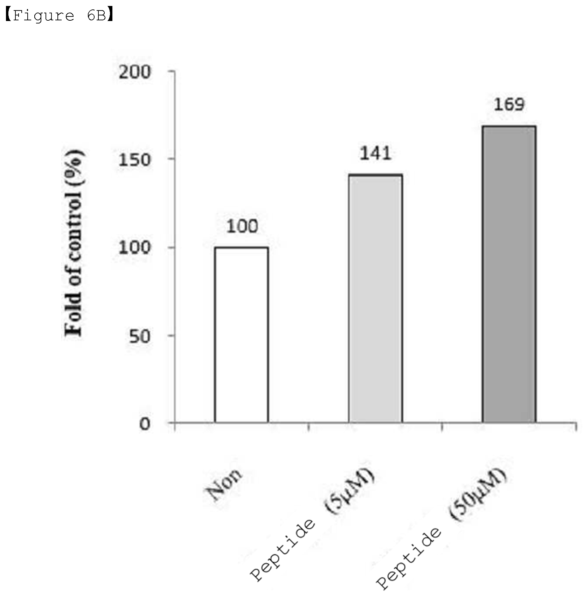

FIGS. 6A and 6B show the results of the peptide of the present invention promoting the differentiation of C2C12 cells and increasing myotube formation. Non is the peptide-untreated group in which the differentiation-induced C2C12 cells were not treated with the peptide.

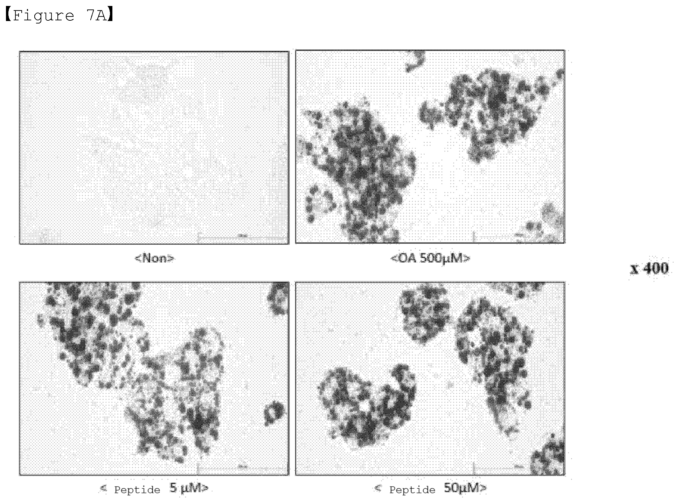

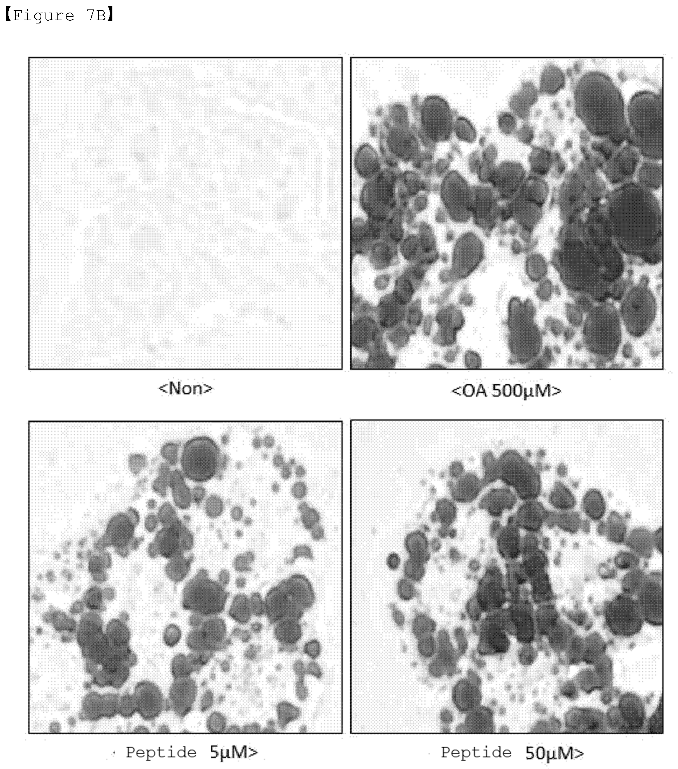

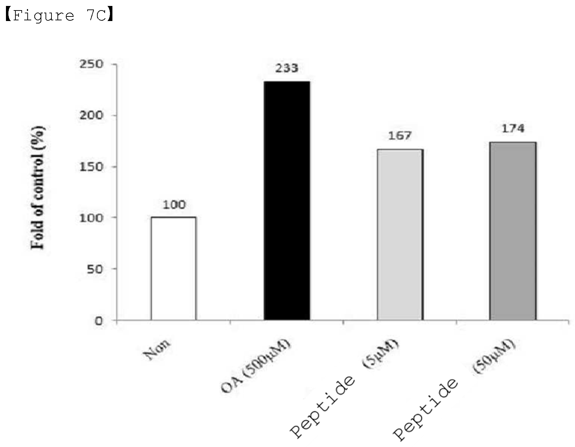

FIGS. 7A, 7B, and 7C show the results of the peptide of the present invention inhibiting fat accumulation in HepG2 hepatocytes. Non is the untreated group in which HepG2 hepatocytes were not treated with oleic acid and the peptide. OA is the oleic acid treated group.

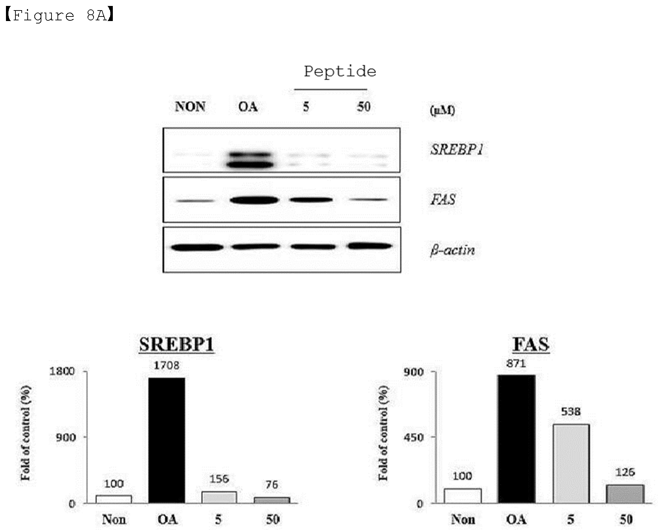

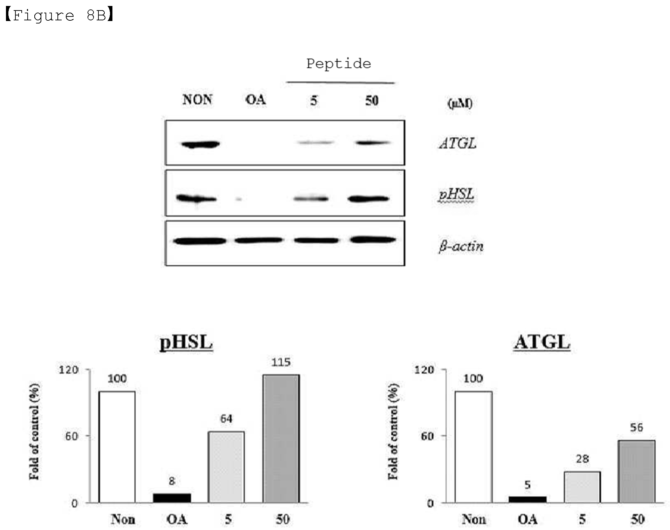

FIGS. 8A and 8B show the results that the peptide of the present invention inhibits the expression of adipogenesis factors SREBP1 and FAS and promotes the expression of lipolysis factors ATGL and pHSL in HepG2 hepatocytes in which lipid accumulation is induced by oleic acid treatment. Non is the untreated group in which HepG2 hepatocytes were not treated with oleic acid and the peptide. OA is the oleic acid treated group.

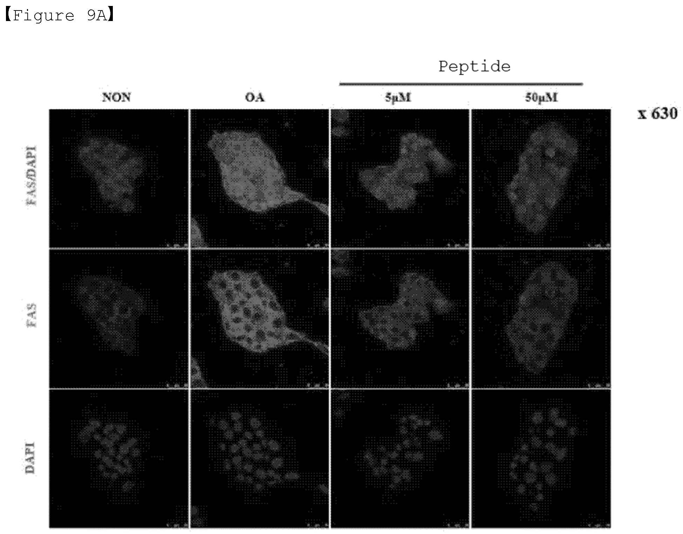

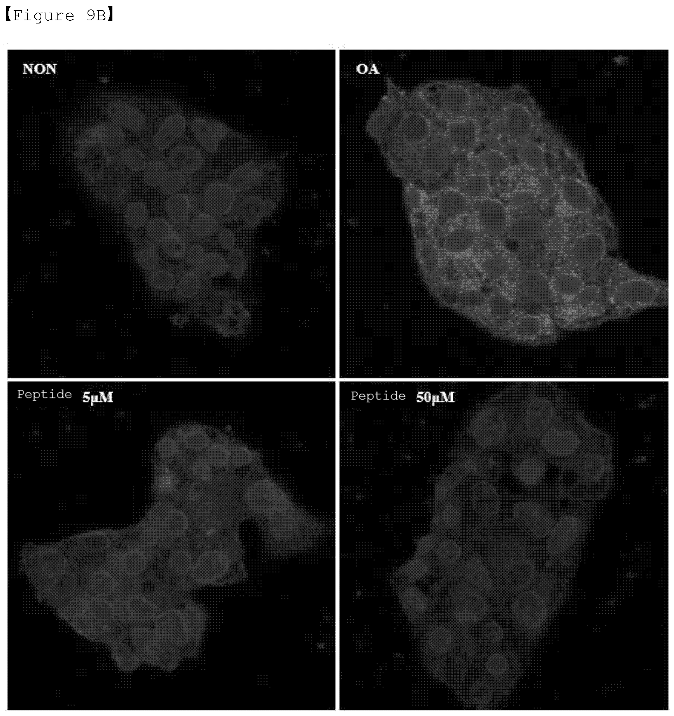

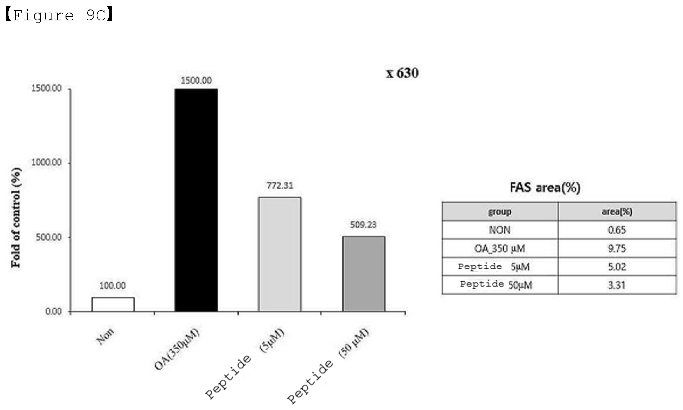

FIGS. 9A, 9B, and 9C show that the peptide of the present invention has activity of inhibiting the increase again in FAS expression induced by oleic acid in HepG2 cells. Non is the untreated group in which HepG2 hepatocytes were not treated with oleic acid and the peptide. OA is the oleic acid treated group.

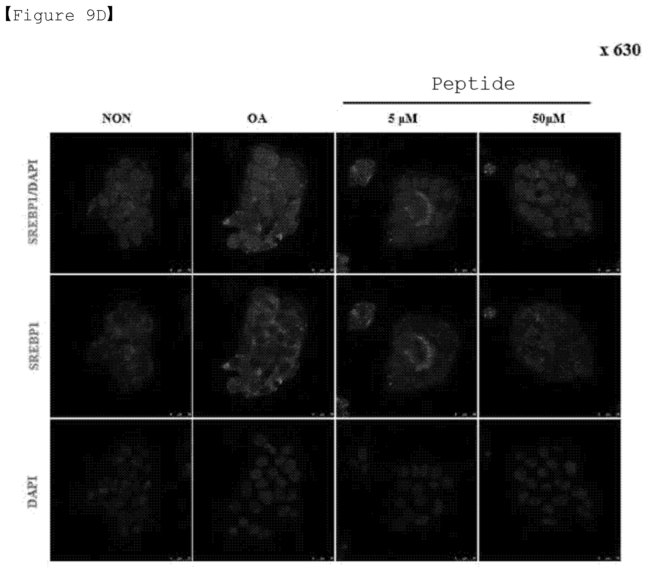

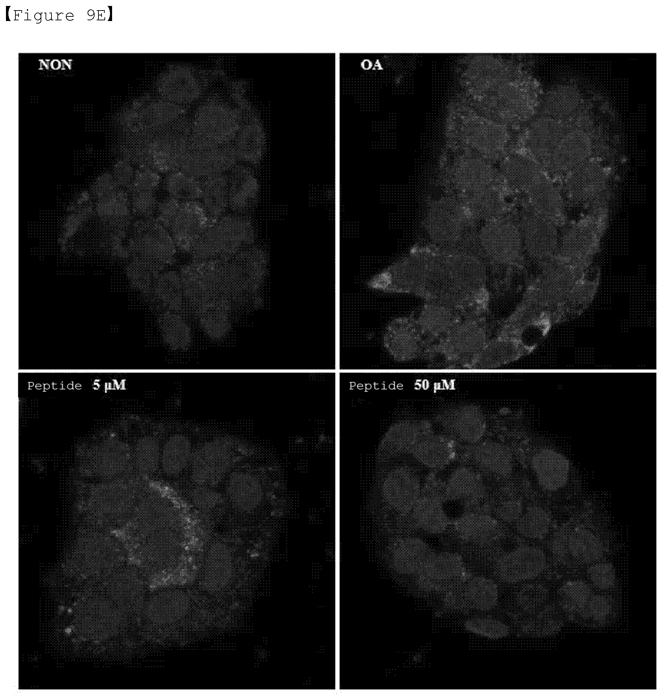

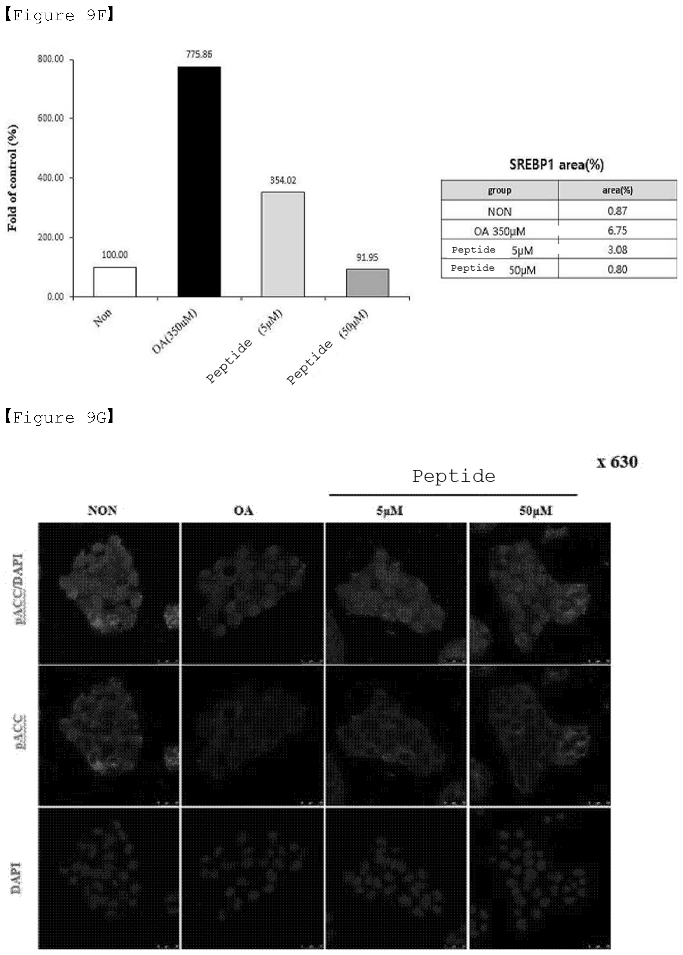

FIGS. 9D, 9E, and 9F show that the peptide of the present invention has activity of inhibiting again the increase in expression of SREBP1 induced by oleic acid in HepG2 cells. Non is the untreated group in which HepG2 hepatocytes were not treated with oleic acid and the peptide. OA is the oleic acid treated group.

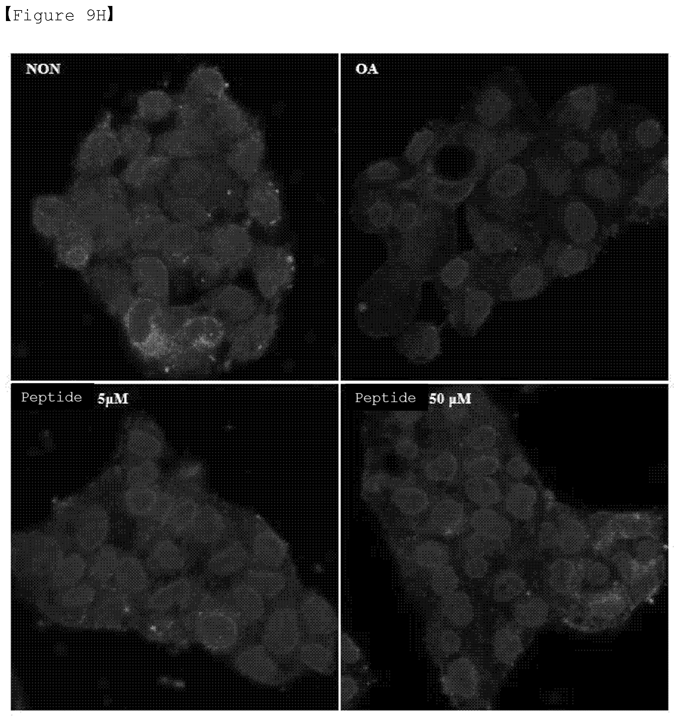

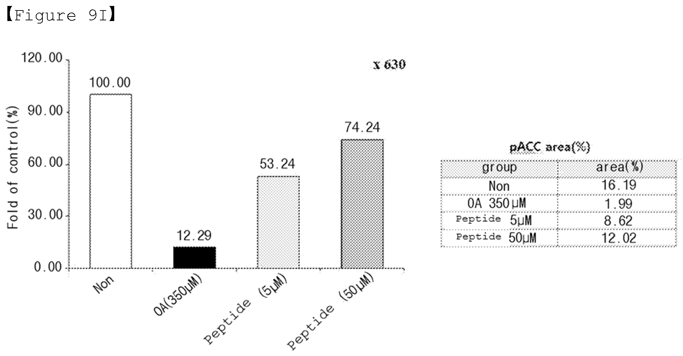

FIGS. 9G, 9H, and 9I show that the peptide of the present invention has the activity of increasing again the decrease in pACC expression induced by oleic acid in HepG2 cells. Non is the untreated group in which HepG2 hepatocytes were not treated with oleic acid and the peptide. OA is the oleic acid treated group.

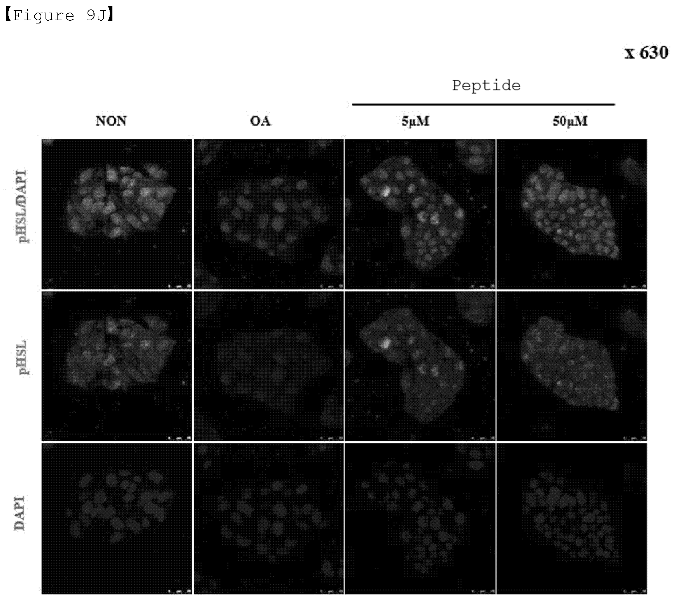

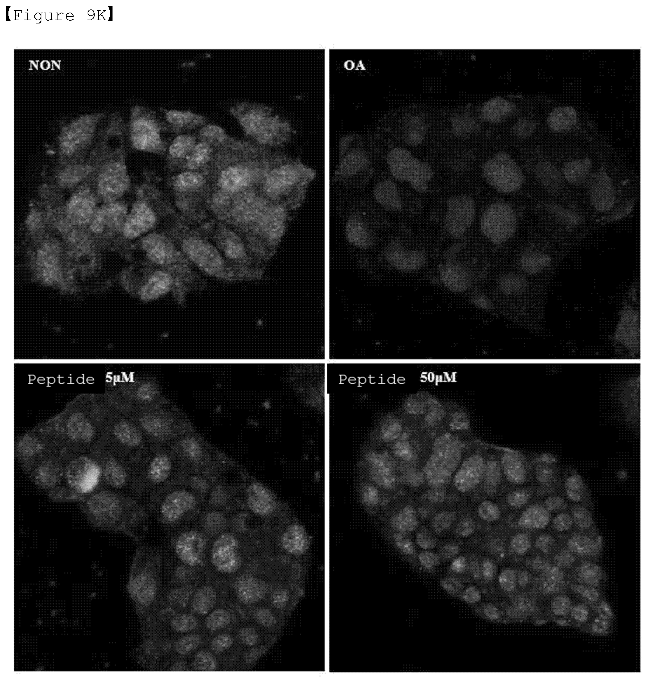

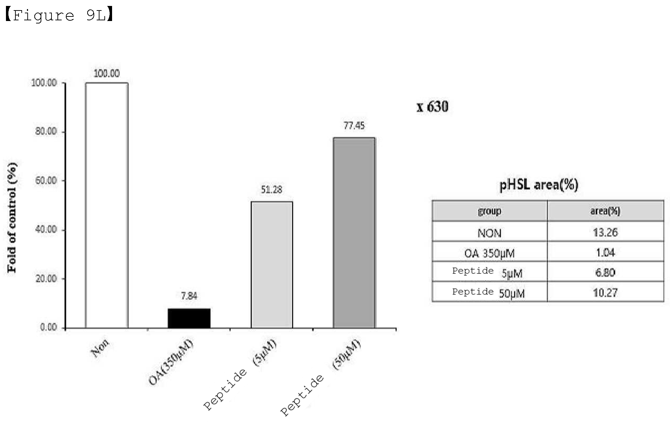

FIGS. 9J, 9K, and 9L show that the peptide of the present invention has the activity of increasing again the decrease in pHSL expression induced by oleic acid in HepG2 cells. Non is the untreated group in which HepG2 hepatocytes were not treated with oleic acid and the peptide. OA is the oleic acid treated group.

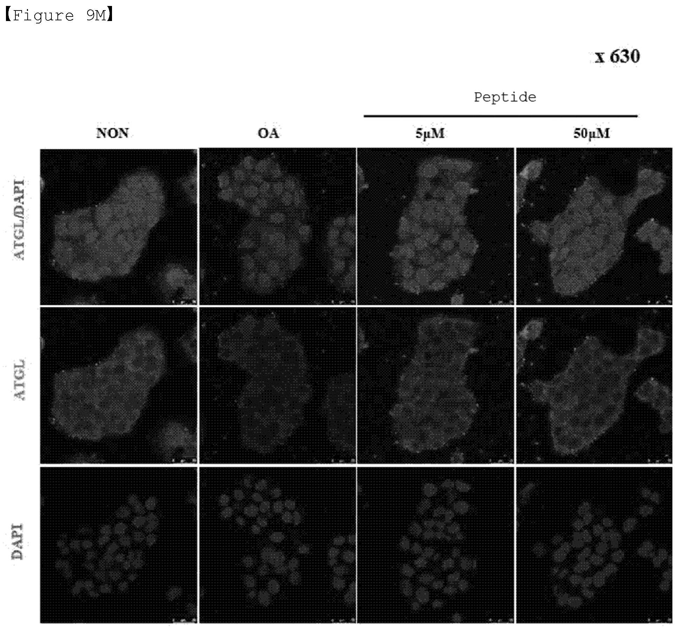

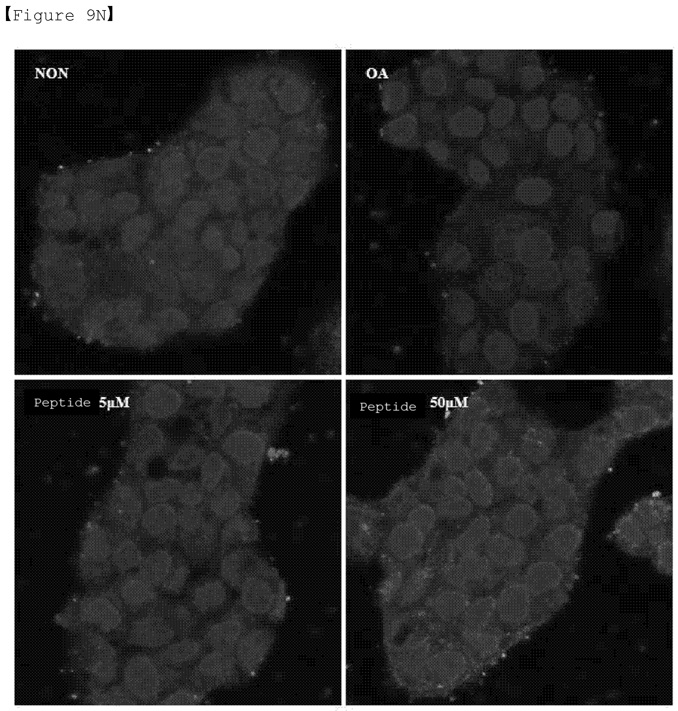

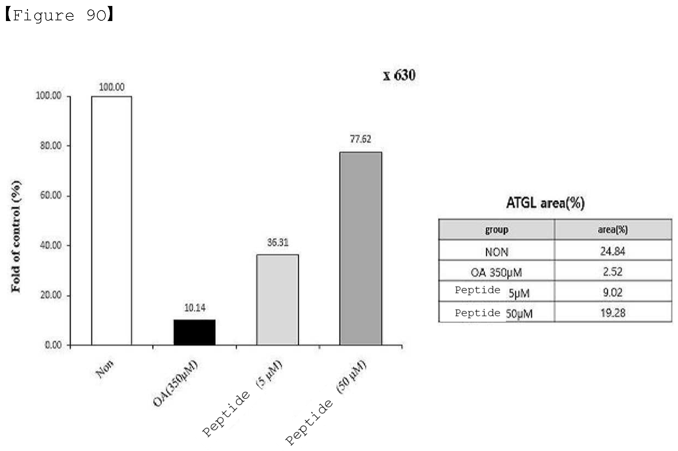

FIGS. 9M, 9N, and 9O show that the peptide of the present invention has the activity of increasing again the decrease in ATGL expression induced by oleic acid in HepG2 cells. Non is the untreated group in which HepG2 hepatocytes were not treated with oleic acid and the peptide. OA is the oleic acid treated group.

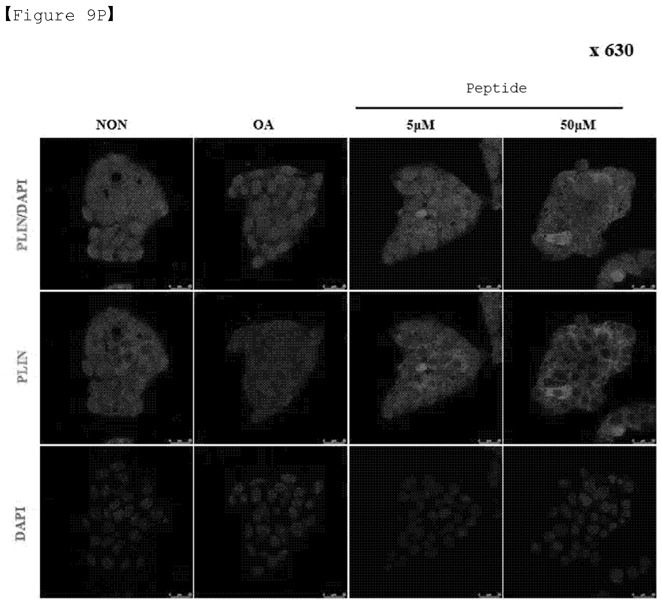

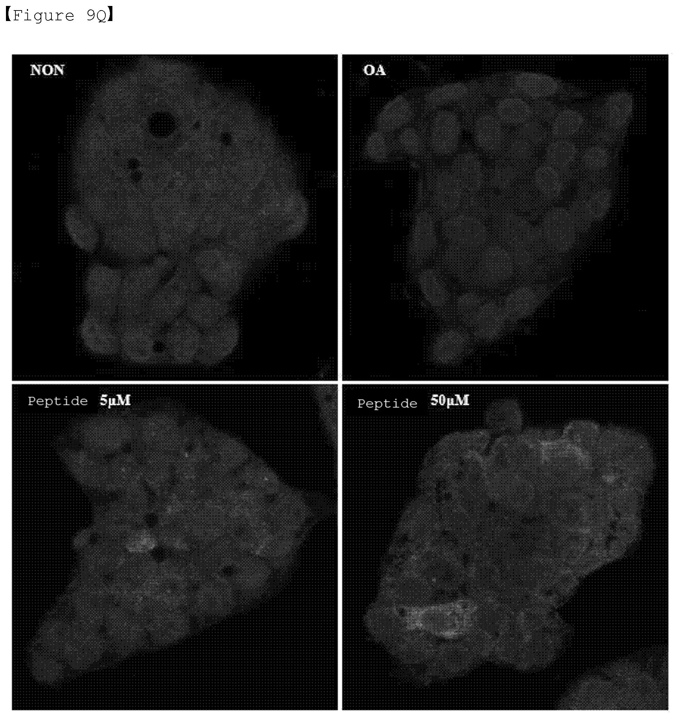

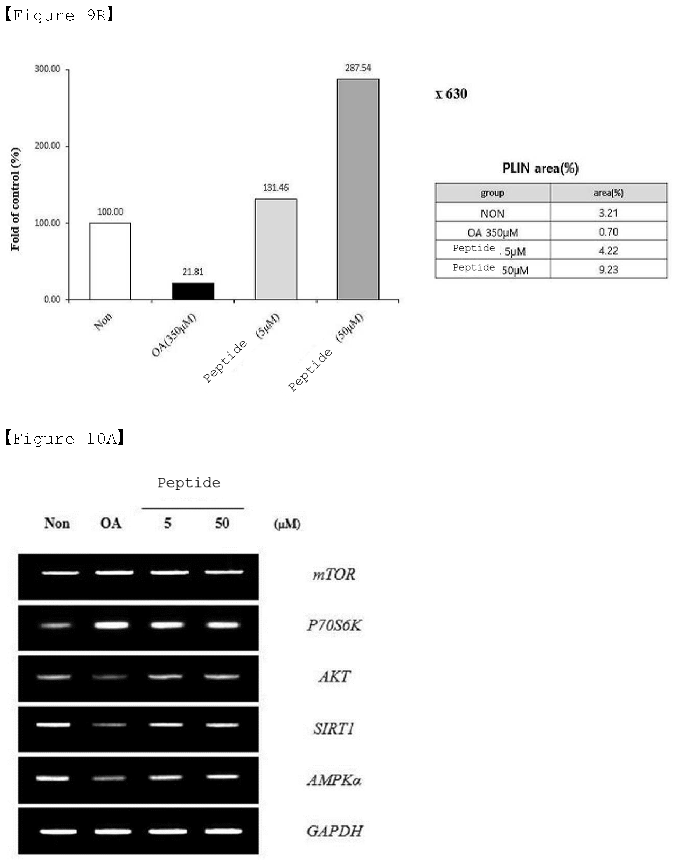

FIGS. 9P, 9Q, and 9R show that the peptide of the present invention has the activity of increasing again the decrease in PLIN expression induced by oleic acid in HepG2 cells. Non is the untreated group in which HepG2 hepatocytes were not treated with oleic acid and the peptide. OA is the oleic acid treated group.

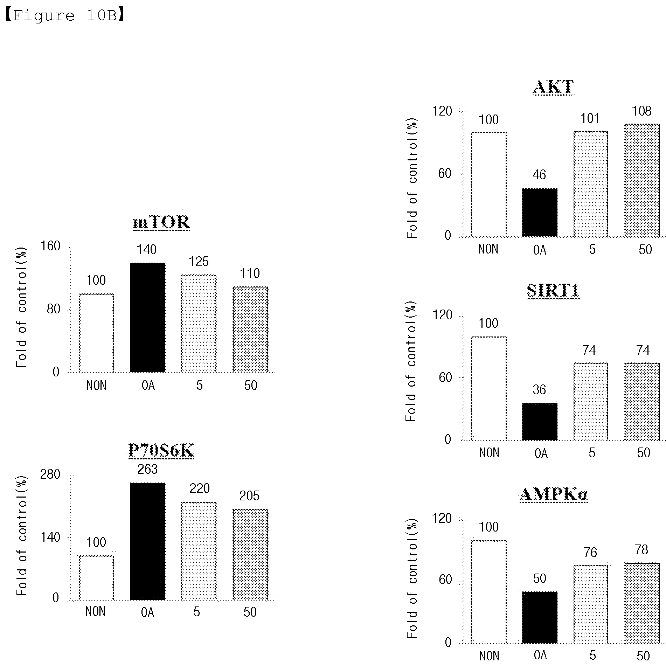

FIGS. 10A and 10B show the results that the peptide of the present invention reduces the expression levels of the insulin resistance factors mTOR and P70S6K genes and increases the expression levels of the insulin sensitivity factors AKT, SIRT1, and AMPKα genes in HepG2 cells treated with oleic acid and having insulin resistance conditions. Non is the untreated group in which HepG2 hepatocytes were not treated with oleic acid and the peptide. OA is the oleic acid treated group.

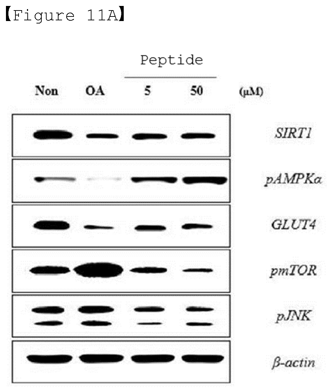

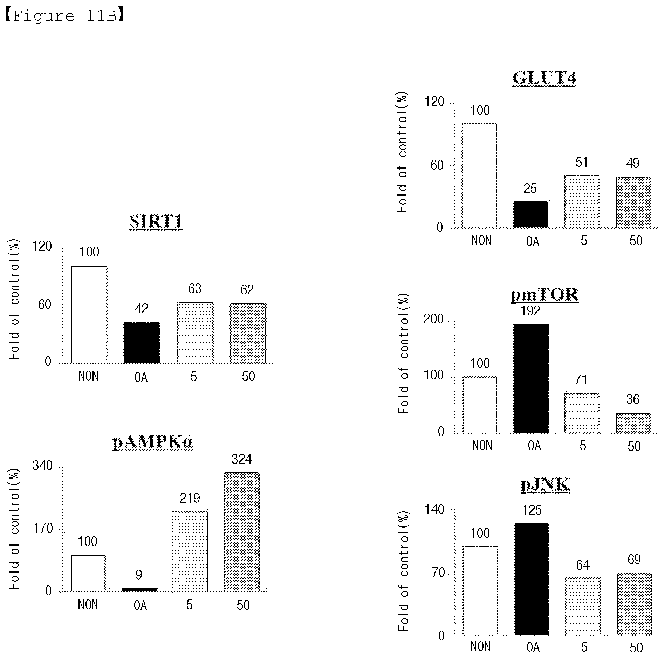

FIGS. 11A and 11B show the results that the peptide of the present invention reduces the expression levels of insulin resistance factors pmTOR and pJNK proteins and increases the expression levels of insulin sensitivity factors pAMPKα, GLUT4, and SIRT1 proteins in HepG2 cells treated with oleic acid and having insulin resistance conditions. Non is the untreated group in which HepG2 hepatocytes were not treated with oleic acid and the peptide. OA is the oleic acid treated group.

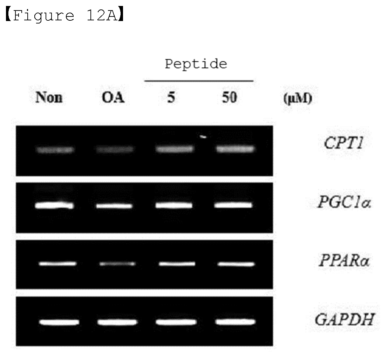

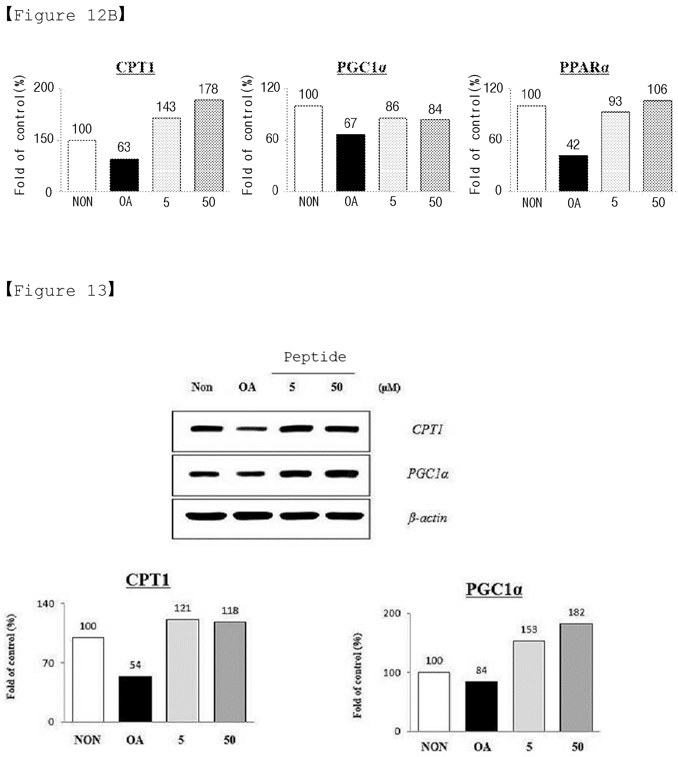

FIGS. 12A and 12B show that the peptide of the present invention increases the expression of genes CPT1, PGC1α, and PPARα, which are factors related to fatty acid oxidation, in HepG2 cells treated with oleic acid and having insulin resistance conditions. Non is the untreated group in which HepG2 hepatocytes were not treated with oleic acid and the peptide. OA is the oleic acid treated group.
FIG. 13 shows that the peptide of the present invention increases the expression of fatty acid oxidation-related factors, CPT1 and PGC1α proteins, in HepG2 cells treated with oleic acid and having fat accumulation conditions. Non is the untreated group in which HepG2 hepatocytes were not treated with oleic acid and the peptide. OA is the oleic acid treated group.

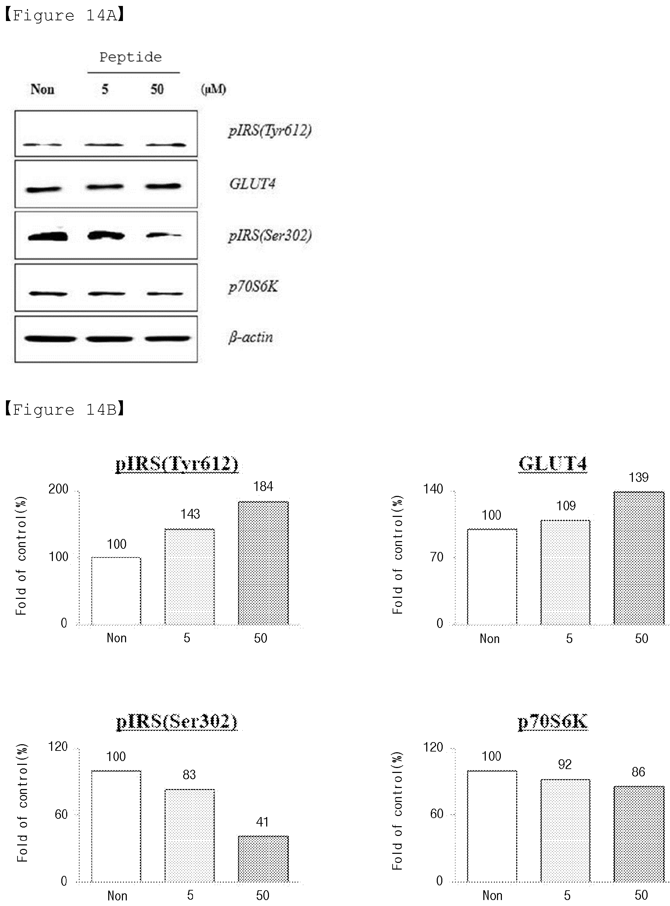

FIGS. 14A and 14B show that the peptide of the present invention increases the protein expression levels of the insulin sensitivity factors pIRS(Tyr612) and GLUT4 and decreases the protein expression levels of the insulin resistance factors pIRS(Ser302) and p70S6K in 3T3-L1 cells. Non is the untreated group of 3T3-L1 cells that were not treated with the peptide.

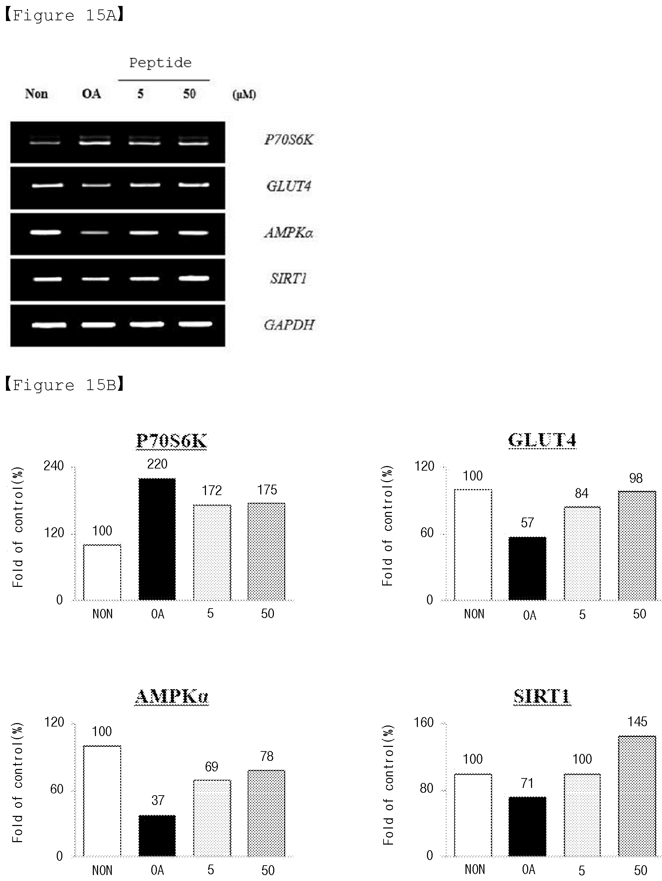

FIGS. 15A and 15B show that the peptide of the present invention reduces the expression level of the insulin resistance factor P70S6K gene and increases the expression levels of the insulin sensitivity factors AMPKα, GLUT4, and SIRT1 genes in oleic acid-treated 3T3-L1 cells. Non is the untreated group in which 3T3-L1 cells were not treated with oleic acid and the peptide. OA is the oleic acid treated group.

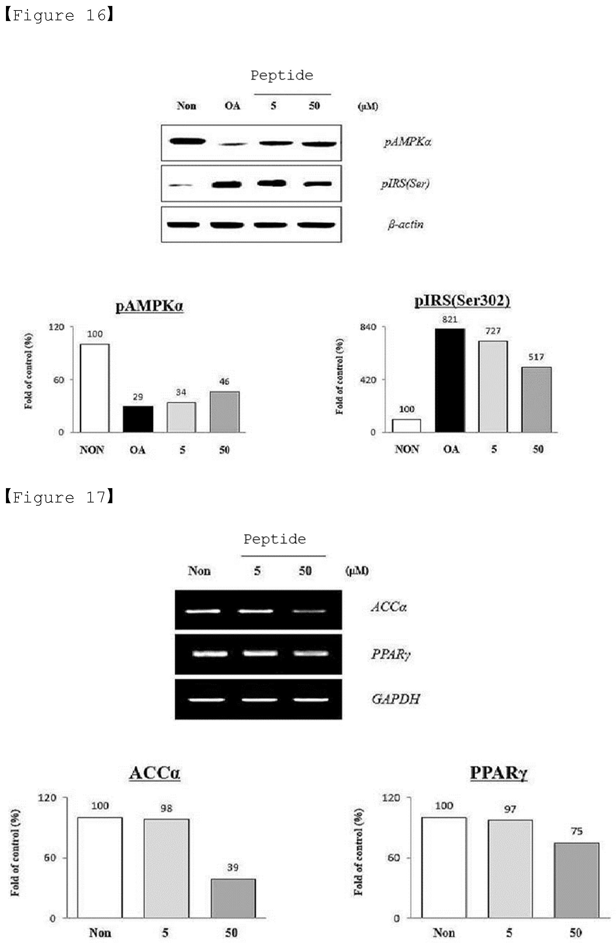

FIG. 16 shows that the peptide of the present invention increases the expression level of the insulin sensitivity factor pAMPKα and decreases the expression level of the insulin resistance factor pIRS (Ser302) in oleic acid-treated 3T3-L1 cells (FIG. 16). Non is the untreated group in which 3T3-L1 cells were not treated with oleic acid and the peptide. OA is the oleic acid treated group.
FIG. 17 shows that the peptide of the present invention reduces the gene expression of adipogenesis-related factors, ACCα and PPAR-γ, in 3T3-L1 cells. Non is the untreated group in which 3T3-L1 cells were not treated with the peptide.

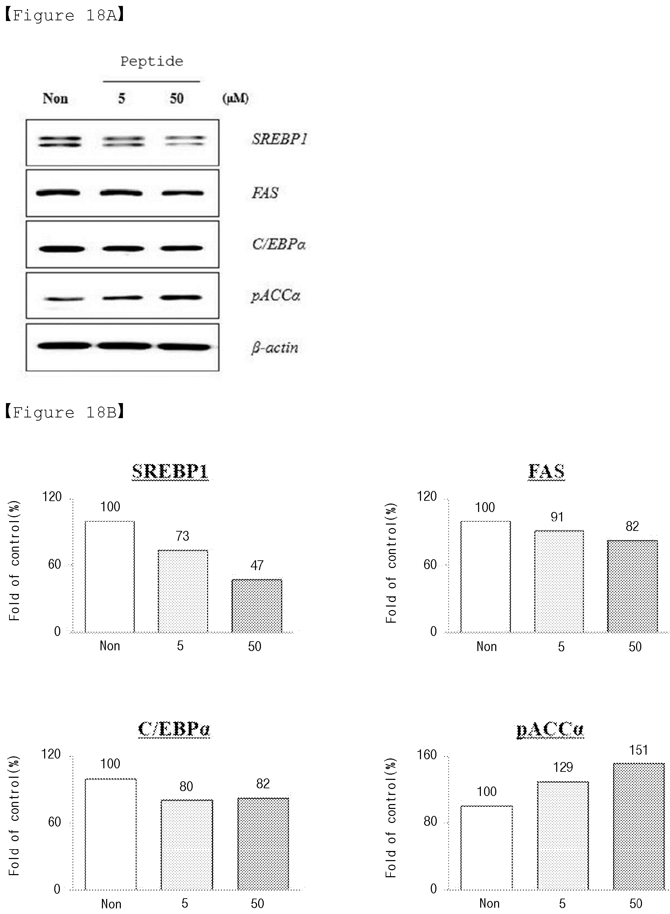

FIGS. 18A and 18B show that the peptide of the present invention reduces the expression of SREBP1, FAS, and C/EBPα, which are adipogenesis-related factors, in 3T3-L1 cells, and increases the expression of pACCα, which promotes the degradation of fatty acids. Non is the untreated group in which 3T3-L1 cells were not treated with the peptide.

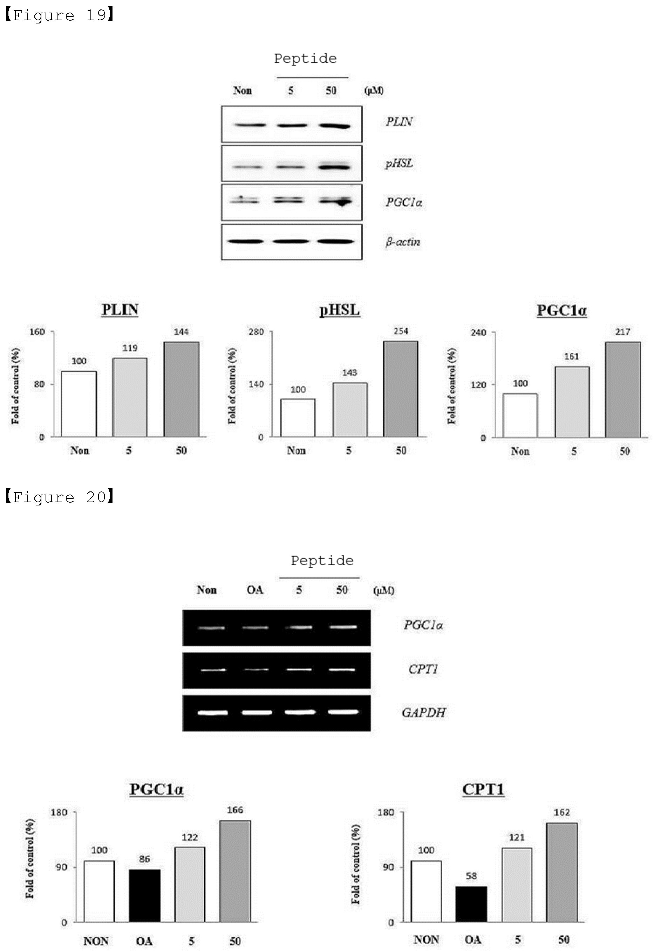

FIG. 19 shows that the peptide of the present invention increases the expression of lipolysis-related factors, PLIN, pHSL, and PGC1α, in 3T3-L1 cells. Non is the untreated group in which 3T3-L1 cells were not treated with the peptide.
FIG. 20 shows that the peptide of the present invention increases the gene expression of fatty acid oxidation-related factors PGC1α and CPT1 in oleic acid-treated 3T3-L1 cells. Non is the untreated group in which 3T3-L1 cells were not treated with oleic acid and the peptide. OA is the oleic acid treated group.

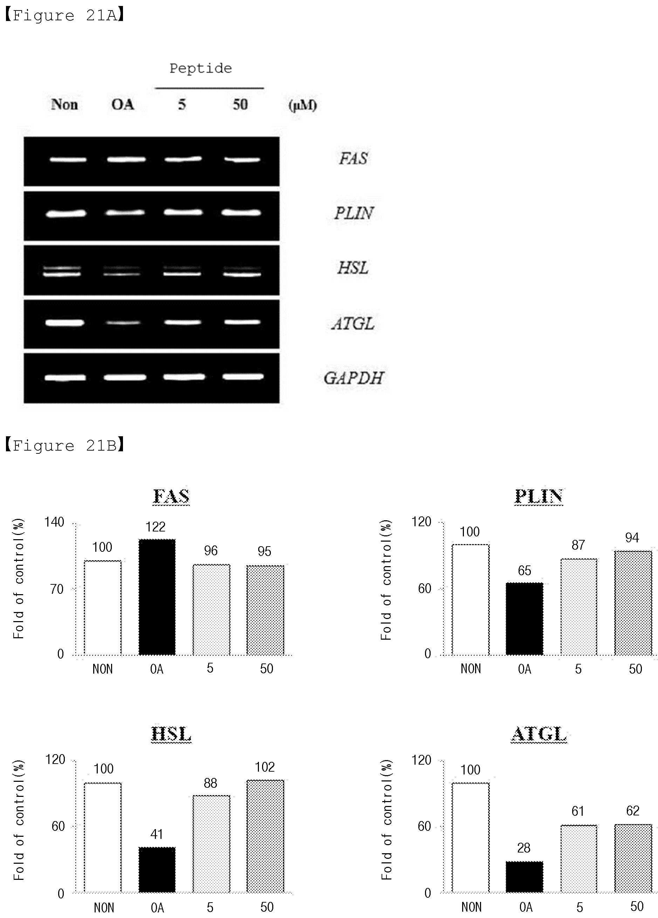

FIGS. 21A and 21B show that the peptide of the present invention reduces the expression of the fatty acid synthesis-related factor FAS gene and increases the expression of the lipolysis-related factors PLIN, HSL, and ATGL genes in oleic acid-treated 3T3-L1 cells. Non is the untreated group in which 3T3-L1 cells were not treated with oleic acid and the peptide. OA is the oleic acid treated group.

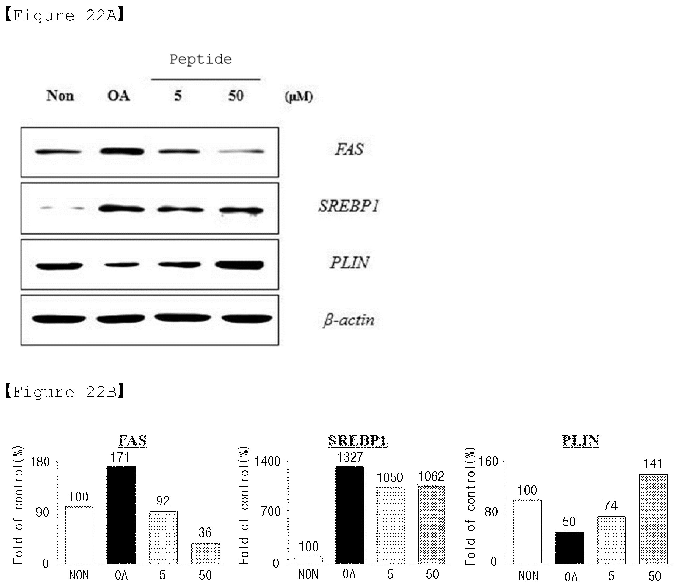

FIGS. 22A and 22B show that the peptide of the present invention reduces the expression of fatty acid synthesis-related factor FAS protein and increases the expression of lipolysis-related factors SREBP1 and PLIN proteins, in oleic acid-treated 3T3-L1 cells. Non is the untreated group in which 3T3-L1 cells were not treated with oleic acid and the peptide. OA is the oleic acid treated group.

### [Best Mode]

Hereinafter, the present invention will be described in detail by way of examples. However, the following examples are intended to specifically illustrate the present invention, and the scope of the present invention is not limited by the following examples.

### Preparation Example 1: Preparation of peptide

A peptide having the amino acid sequence SEQ ID NO: 1 listed in Table 1 below was synthesized using an automated peptide synthesizer (Milligen 9050, Millipore, USA), and the synthesized peptides were purified using C18 reverse-phase high-performance liquid chromatography (HPLC) (Waters Associates, USA). An ACQUITY UPLC BEH300 C18 column (2.1 mm × 100 mm, 1.7 µm, Waters Co, USA) was used.

**[Table 1]**

| SEQ ID NO | Amino acid sequence (N-terminus → C-terminus) |
|---|---|
| 1 | HGTY (His-Gly-Thr-Tyr) |

The efficacy of the peptide of SEQ ID NO: 1 prepared above was evaluated through the following experiment.

### [Test Example 1 - 6] Test Example for Myoblast

### Test Example 1: Analysis of cell proliferation-promoting activity against myoblasts

The effect of the peptide of SEQ ID NO: 1 prepared in Preparation Example 1 on the proliferation ability and cytotoxicity of myoblasts was evaluated.

C2C12 cells (mouse-derived myoblasts) were cultured in DMEM medium containing 1% P/S and 10% FBS. When cell confluency reached 70 to 80%, cells were seeded into 96-well culture plates at a concentration of 5 x 10³ cells/200 µl. On the next day, the medium was replaced with serum-free DMEM (1% P/S), and after 4 hours, it was replaced with DMEM medium containing 0.2% FBS and peptides of different concentrations (0 µM, 3.9 µM, 7.81 µM, 15.63 µM, 31.25 µM, 62.5 µM, 125 µM, 250 µM, 500 µM). At 48 and 72 hours after replacement, the reaction was carried out using EZ-cytox (DoGenBio Co., Ltd, Seoul, Korea) until the absorbance of Non (no peptide added) reached a value of 1.0, and the absorbance was measured at 450 nm using a Spectrophotometer (SPECTRAMAX M2e). When C2C12 cells were treated with peptides at different concentrations for 24 and 48 hours, it was confirmed that there was no significant difference in concentration for 24-hour treatment, but cell proliferation increased in a peptide concentration-dependent manner for 48-hour treatment (FIG. 1).

### Test Example 2: Analysis of expression of proliferation-related marker genes in myoblasts

The effect of the peptide of SEQ ID NO: 1 prepared in Preparation Example 1 on the proliferation ability of myoblasts was evaluated by analyzing the expression of proliferation-related marker genes.

C2C12 cells (mouse-derived myoblasts) were cultured in DMEM medium containing 1% P/S and 10% FBS. When cell confluency reached 70 to 80%, cells were seeded into 6-well culture plates at a concentration of 5 x 10⁵ cells/2 ml. On the next day, the medium was replaced with serum-free DMEM (1% P/S), and after about 4 hours, was treated with peptides (5 µM, 50 µM), and one hour after peptide treatment, the cells were harvested and RNA was extracted using Trizol (Thermo Fisher Scientific, USA). DNA complementary to the extracted RNA was synthesized using TOPScript^{™} RT DryMIX (enzynomics, Korea), and then polymerase chain reaction (PCR) was performed on the genes of factors (markers) involved in cell proliferation using TOPsimple^{™} DryMIX-nTaq (enzynomics, Korea). Next, the reaction products were run on a 1.5% agarose gel to compare the mRNA expression levels of the aforementioned factors (markers) for each sample. The primer sequences for the genes used in the PCR are listed in Table 2 below, and the GAPDH gene was used as a control group.

**[Table 2]**

| Primer Name | Sequence (5'-> 3') | SEQ ID NO |
|---|---|---|
| PCNA Forward | AGC GGA GAA GGT GCT GCA G | 2 |
| PCNA Reverse | ATA GCG GCG GTA TGT GTC GG | 3 |
| NDRG2 Forward | CAC ACA GAC CTC GTT CCT CA | 4 |
| NDRG2 Reverse | GAT GCT GCA CTG GTC AGA GA | 5 |
| CDK4 Forward | AGT TTC TAA GCG GCC TGG AT | 6 |
| CDK4 Reverse | AAC TTC AGG AGC TCG GTA CC | 7 |
| GAPDH Forward | GTG ATG GCA TGG ACT GTG GT | 8 |
| GAPDH Reverse | GGA GCC AAA AGG GTC ATC AT | 9 |

Experimental results confirmed that the expression of PCNA (Proliferating cell nuclear antigen), NDRG2 (N-myc Downstream-Regulated Gene 2), and CDK4 (Cyclin-dependent kinase 4) genes related to cell proliferation in C2C12 cells was significantly increased by peptide treatment (FIGS. 2A and 2B).

### Test Example 3: Analysis of cell proliferation-related marker protein expression in myoblasts

The effect of the peptide of SEQ ID NO: 1 prepared in Preparation Example 1 on the proliferation ability of myoblasts was evaluated by analyzing the expression of proliferation-related marker proteins.

C2C12 cells (mouse-derived myoblasts) were cultured in DMEM medium containing 1% P/S and 10% FBS. When cell confluency reached 70 to 80%, cells were seeded into 6-well culture plates at a concentration of 5 x 10⁵ cells/2 ml. On the next day, the medium was replaced with serum-free DMEM (1% P/S), and after about 4 hours, was treated with peptides (5 µM, 50 µM). One hour after peptide treatment, cell lysis buffer was added to lyse the cells, and then the proteins were obtained by centrifuging at 4°C and 12,000 rpm for 30 minutes. The obtained proteins were quantified using a BCA kit. The protein was electrotransferred onto a membrane after performing SDS-PAGE (sodium dodecyl sulfate-polyacrylamide gel electrophoresis). The membrane to which the protein was attached was blocked by treatment with 5% skim milk, and then treated with the primary antibody and reacted overnight at 4°C. Anti-SIRT1 antibody (Cell signaling Technology, USA), anti-Ki67 antibody (Santa Cruz, USA), anti-Pax7 antibody (Santa Cruz, USA), anti-pAKT antibody (Cell signaling Technology, USA), and anti-β-actin antibody (Santa Cruz, USA) were used as primary antibodies. After washing the reaction mixture with PBS-T, secondary antibodies (Peroxidase-conjugated AffiniPure Goat Anti-Rabbit IgG (H+L), Jackson immunoResearch, USA; Peroxidase-conjugated AffiniPure Goat Anti-Mouse IgG (H+L), Jackson immunoResearch, USA) were reacted at room temperature for 1 hour and were washed again with PBS-T, the mixture was visualized using Gel Doc (Bio-Rad, Hercules, CA, USA) by treatment with Western detection reagents (Elpis Biotech, Daejeon, Korea).

As a result of the experiment, when the peptides were treated at concentrations of 5 µM and 50 µM for 1 hour, the expression levels of SIRT1 (Sirtuin1), Ki67, Pax7, and pAKT proteins all increased in a concentration-dependent manner. In particular, the expression level of Pax7 protein increased 2.6-fold and 3.8-fold, respectively, compared to the control group, showing a significant increase (FIGS. 3A and 3B).

### Test Example 4: Analysis of expression of early/mid-differentiation marker genes and signaling genes related to muscle protein synthesis in myoblasts

The effect of the peptide of SEQ ID NO: 1 prepared in Preparation Example 1 on the expression of genes of early and mid-differentiation markers of myoblasts and signaling factors involved in muscle protein synthesis was analyzed.

C2C12 cells (mouse-derived myoblasts) were cultured in DMEM medium containing 1% P/S and 2% BCS (bovine calf serum). When cell confluency reached 70 to 80%, cells were seeded into 6-well culture plates at a concentration of 5 x 10⁵ cells/2 ml. Subsequently, when cell confluency reached 100% in a 6-well culture plate, the differentiation medium (1% P/S, 2% horse serum) and peptides (5µM, 50µM) were replaced. After that, the differentiation medium (1% P/S, 2% horse serum) and peptides (5µM, 50µM) were replaced every two days, and cells were harvested three days after the first replacement and RNA was extracted using Trizol (Thermo Fisher Scientific, USA). The extracted RNA was used to synthesize DNA complementary to the RNA using TOPScript^{™} RT DryMIX (enzynomics, Korea), and then polymerase chain reaction (PCR) was performed on genes of early and mid-differentiation markers and markers involved in signaling involved in muscle protein synthesis using TOPsimple^{™} DryMIX-nTaq (enzynomics, Korea). Next, the reaction products were run on a 1.5% agarose gel to compare the mRNA expression levels of the markers for each sample. The primer sequences for the genes used in the PCR are listed in Table 3 below, and the GAPDH gene was used as a control group.

**[Table 3]**

| Primer Name | Sequence (5'-> 3') | SEQ ID NO |
|---|---|---|
| mTOR Forward | TTG AGG TCG CTA TGA CCA GAG AGA A | 10 |
| mTOR Reverse | TTA CCA GAA GGG ACA CCA GCC AAT G | 11 |
| Myf6 Forward | ATC AGC TAC ATT GAG CGT CTA CA | 12 |
| Myf6 Reverse | CCT GGA ATG ATC CGA AAC ACT TG | 13 |
| Myf5 Forward | TAT GAA GGC TCC TGT CC | 14 |
| Myf5 Reverse | ACG TGC TCC TCA TCG TCT G | 15 |
| Myf4 Forward | ATC AGC TAC ATT GAG CGT CTA CA | 16 |
| Myf4 Reverse | CCT GGA ATG ATC CGA AAC ACT TG | 17 |
| MyoD Forward | AGT GAA TGA GGC CTT CGA GA | 18 |
| MyoD Reverse | CTG GGT TCC CTG TTC TGT GT | 19 |
| GAPDH Forward | GTG ATG GCA TGG ACT GTG GT | 8 |
| GAPDH Reverse | GGA GCC AAA AGG GTC ATC AT | 9 |

According to the above experimental method, differentiation was induced in C2C12 myoblasts with 2% horse serum, and at the same time the peptide was treated for 3 days to confirm the effect of the peptide on differentiation. RT-PCR experimental results showed that the expression levels of MyoD and Myf6, which are factors involved in mid and late-differentiation, increased upon peptide treatment. In particular, Myf6 showed a significant increase, with expression increasing 1.8-fold compared to the control group upon treatment with 50 µM of peptide. Additionally, it was confirmed that the expression levels of mTOR, Myf5, and Myf4, which are factors involved in early and mid-differentiation, also increased in a concentration-dependent manner (FIGS. 4A and 4B).

### Test Example 5: Analysis of expression of mid and late-differentiation markers and signaling proteins involved in muscle protein synthesis in myoblasts

The effect of the peptide of SEQ ID NO: 1 prepared in Preparation Example 1 on the expression of myoblast differentiation markers during the mid and late-stages and signaling proteins involved in muscle protein synthesis was analyzed.

C2C12 cells (mouse-derived myoblasts) were cultured in DMEM medium containing 1% P/S and 2% BCS (bovine calf serum). When cell confluency reached 70 to 80%, cells were seeded into 6-well culture plates at a concentration of 5 x 10⁵ cells/2 ml. Subsequently, when cell confluency reached 100% in a 6-well culture plate, the differentiation medium (1% P/S, 2% horse serum) and peptide (5 µM, 50 µM) were replaced. Afterward, the differentiation medium (1% P/S, 2% horse serum) and peptide (5 µM, 50 µM) were replaced every two days, and lysis buffer was added to lyse the cells three days after the first replacement. Subsequently, the cell lysate was centrifuged at 4°C and 12,000 rpm for 30 minutes to obtain the protein. The obtained protein was quantified using a BCA kit. The protein was electrotransferred onto a membrane after performing SDS-PAGE (sodium dodecyl sulfate-polyacrylamide gel electrophoresis). After blocking the protein-attached membrane with 5% skim milk, the membrane was treated with the primary antibody and reactioned overnight at 4°C. The primary antibodies used are as follows: anti-α-actinin antibody (Santa Cruz, USA); anti-MyoG antibody (Santa Cruz, USA); anti-Myf6 antibody (Abcam, UK); anti-SIRT1 antibody (Cell Signaling Technology, USA); anti-pAMPKα antibody (Cell Signaling Technology, USA); anti-pAKT antibody (Cell Signaling Technology, USA); anti-pmTOR antibody (Cell Signaling Technology, USA); anti-p70S6 Kinase antibody (Cell Signaling Technology, USA); anti-α-tubulin antibody (Santa Cruz, USA). The reactant was washed with PBS-T, then reacted with secondary antibodies (Peroxidase-conjugated AffiniPure Goat Anti-Rabbit IgG (H+L), Jackson immunoResearch, USA; Peroxidase-conjugated AffiniPure Goat Anti-Mouse IgG (H+L), Jackson immunoResearch, USA) at room temperature for 1 hour, washed again with PBS-T, and then visualized by treatment with a Western detection reagent (Elpis Biotech, Daejeon, Korea) using Gel Doc (Bio-Rad, Hercules, CA, USA).

As a result of the experiment, the expression of differentiation markers α-actinin, Myf6, and MyoG proteins increased in a concentration-dependent manner upon peptide treatment (FIGS. 5A and 5B). In addition, the expression of signaling proteins involved in muscle protein synthesis, such as SIRT1, pAMPKα, pAKT, pmTOR, and p70S6K, also increased in a concentration-dependent manner upon peptide treatment (FIGS. 5C and 5D).

### Test Example 6 : Microscopic analysis of the morphology of differentiation-induced myoblasts

The effect of the peptide of SEQ ID NO: 1 prepared in Preparation Example 1 on the synthesis of myotubes in myoblasts was analyzed through morphological observation using a microscope.

C2C12 cells (mouse-derived myoblasts) were cultured in DMEM medium containing 1% P/S and 2% BCS (bovine calf serum). When cell confluency reached approximately 80%, cells were seeded into 6-well culture plates at a concentration of 5 x 10⁵ cells/2 ml. Subsequently, when cell confluency reached 100% in a 6-well culture plate, the differentiation medium (1% P/S, 2% horse serum) and peptides (5µM, 50µM) were replaced. After that, the differentiation medium (1% P/S, 2% horse serum) and peptides (5µM, 50µM) were replaced every two days, and images of the cells were taken using an optical microscope (FLEXACAM C1, Leica, Germany) three days after differentiation induction.

The above experiment aimed to determine whether cell differentiation is promoted when peptides are treated simultaneously with differentiation induction in C2C12 myoblasts. Cell morphology was examined using a microscope, and the thickness of the formed myotubes was measured. The experimental results showed that myoblast differentiation was further promoted when peptides were treated compared to the control group (differentiation induction, Non), and that a large number of long and thick myotubes were formed when the peptides were treated at high concentrations (FIGS. 6A and 6B).

### [Test Examples 7 to 13] Test Example for hepatocytes

### Test Example 7: Analysis of fat accumulation in hepatocytes

The effect of the peptide of SEQ ID NO: 1 prepared in Preparation Example 1 on fat accumulation in hepatocytes (hepatocytes) was analyzed.

HepG2 cells (human-derived hepatocytes) were cultured in DMEM medium containing 1% P/S and 10% FBS. When cell confluency reached approximately 70 to 80%, cells were seeded into a 12-well culture plate at a concentration of 1 x 10⁵ cells/1 ml. Subsequently, when cell confluency reached approximately 70 to 80% in a 12-well culture plate, the medium was replaced with serum-free DMEM. After 4 hours, lipid accumulation was induced by replacing the medium with serum-free DMEM (1% BSA) supplemented with 500 µM oleic acid (Sigma, St. Louis, MO, USA). After inducing lipid accumulation for 24 hours, the medium was replaced with serum-free DMEM (1% BSA) supplemented with 500 µM oleic acid (Sigma, St. Louis, MO, USA) and peptides (5 µM, 50 µM), and cultured for 48 hours. To compare the degree of adipogenesis, the cells were washed twice with PBS and fixed with 10% formaldehyde. Oil-Red O solution (Sigma, USA) was added to the fixed cells to stain lipid globules for 2 hours, after which they were washed with distilled water (DW) and observed under a microscope. Subsequently, the distilled water was removed, the cells were dried, washed with isopropanol, and the absorbance was measured using a Spectrophotometer (SPECTRAMAX M2e) at an OD wavelength of 540 nm.

As a result of the experiment, it was observed that lipogenesis increased in HepG2 hepatocytes due to oleic acid treatment, and lipogenesis was inhibited due to peptide treatment (FIGS. 7A, 7B, and 7C).

### Test Example 8: Analysis of expression of proteins related to adipogenesis and lipolysis in hepatocytes

The effect of the peptide of SEQ ID NO: 1 prepared in Preparation Example 1 on the expression of proteins related to adipogenesis and lipolysis in hepatocytes was analyzed.

HepG2 cells (human-derived hepatocytes) were cultured in DMEM medium comprising 1% P/S and 10% FBS. When cell confluency reached approximately 70 to 80%, cells were seeded into a 12-well culture plate at a concentration of 1 x 10⁵ cells/1 ml. Subsequently, when cell confluency reached approximately 70 to 80% in a 12-well culture plate, the medium was replaced with serum-free DMEM. After 4 hours, lipid accumulation was induced by replacing the medium with serum-free DMEM (1% BSA) supplemented with 500 µM oleic acid (Sigma, St. Louis, MO, USA). After inducing fat accumulation for 24 hours, the cells were cultured for 48 hours with serum-free DMEM (1% BSA) supplemented with 500 µM oleic acid (Sigma, St. Louis, MO, USA) and peptides (5 µM, 50 µM). After culture, lysis buffer was added to lyse the cells, and the cell lysate was centrifuged at 4°C and 12,000 rpm for 30 minutes to obtain the protein. The obtained protein was quantified using a BCA kit. The protein was electrotransferred onto a membrane after performing SDS-PAGE (sodium dodecyl sulfate-polyacrylamide gel electrophoresis). After blocking the protein-attached membrane with 5% skim milk, the membrane was treated with the primary antibody and reacted overnight at 4°C. The primary antibodies used were as follows: anti-SREBP1 antibody (Abcam, UK); anti-FAS antibody (Cell signaling Technology, USA); anti-ATGL antibody (Cell signaling Technology, USA); anti-pHSL antibody (Cell signaling Technology, USA); anti-β-actin antibody (Santa Cruz, USA). The reactant was washed with PBS-T, then the secondary antibodies (Peroxidase-conjugated AffiniPure Goat Anti-Rabbit IgG (H+L), Jackson immunoResearch, USA; Peroxidase-conjugated AffiniPure Goat Anti-Mouse IgG (H+L), Jackson immunoResearch, USA) were reacted at room temperature for 1 hour, washed again with PBS-T, and then visualized using Gel Doc (Bio-Rad, Hercules, CA, USA) by treatment with Western detection reagent (Elpis Biotech, Daejeon, Korea).

Experimental results showed that in HepG2 hepatocytes, oleic acid treatment increased the expression of SREBP1 (Sterol Regulatory Element Binding Protein-1) and FAS (Fatty acid synthase), which are proteins involved in adipogenesis, and that the increased expression of SREBP1 and FAS decreased again when treated with a peptide (FIG. 8A). Additionally, it was confirmed that oleic acid treatment inhibited the expression of ATGL (Adipose triglyceride lipase) and pHSL (Hormone-sensitive lipase), which are proteins involved in lipolysis, in HepG2 hepatocytes, but when treated with a peptide, the decreased expression levels of ATGL and pHSL increased again (FIG. 8B).

### Test Example 9: Immunofluorescence analysis of expression of markers related to adipogenesis and lipolysis in hepatocytes

The effect of the peptide of SEQ ID NO: 1 prepared in Preparation Example 1 on the expression of markers related to adipogenesis and lipolysis in hepatocytes was analyzed by immunofluorescence.

HepG2 cells (human-derived hepatocytes) were cultured in DMEM medium containing 1% P/S and 10% FBS. When cell confluency reached approximately 70 to 80%, cells were seeded into 6-well culture plates at a concentration of 3 x 10⁵ cells/2 ml. Subsequently, when cell confluency in a 6-well culture plate reached approximately 70 to 80%, the medium was replaced with serum-free DMEM. After 4 hours, lipid accumulation was induced by replacing the medium with serum-free DMEM (1% BSA) supplemented with 350 µM oleic acid (Sigma, St. Louis, MO, USA). After inducing lipid accumulation for 24 hours, the cells were replaced with serum-free DMEM (1% BSA) supplemented with 350 µM oleic acid (Sigma, St. Louis, MO, USA) and peptides (5 µM, 50 µM), and cultured for 48 hours. After culture, the cells were fixed with 4% paraformaldehyde (PFA) for 10 minutes at room temperature (RT) and permeated with 0.1% Triton X-100 for 5 minutes. Subsequently, the cells were blocked with PBS, 10% FBS, and 0.1% Triton X-100 for 1 hour at room temperature, then treated with the primary antibody and incubated at room temperature for 1 hour. The primary antibodies used are as follows: anti-FAS antibody (Cell signaling Technology, USA); anti-SREBP1 antibody (Abcam, UK); anti-pACC antibody (Cell signaling Technology, USA); anti-pHSL antibody (Cell signaling Technology, USA); anti-pATGL antibody (Cell signaling Technology, USA); anti-PLIN antibody (Affinity biosciences, USA). After incubation, the cells were washed three times and incubated with a secondary antibody (goat anti-Rabbit IgG H&L, Abcam, USA) at room temperature for 30 minutes. The samples were washed three times, and the nuclei were counterstained with DAPI (Santa Cruz, USA). Images were acquired using a LEICA fluorescence microscope (TCS SP8, LEICA, Germany).

Regarding the expression level of FAS (Fatty acid synthase), an enzyme involved in fatty acid synthesis, it was confirmed that FAS expression increased when lipid accumulation was induced with oleic acid in HepG2 cells, and when the cells were treated with a peptide, the increased FAS expression level decreased again in a concentration-dependent manner (FIGS. 9A, 9B and 9C).

Regarding the expression level of SREBP1 (Sterol Regulatory Element Binding Protein-1), which induces the synthesis of lipids and cholesterol in the liver, it was confirmed that SREBP1 expression increased when lipid accumulation was induced with oleic acid in HepG2 cells, and when the cells were treated with a peptide, the increased expression level of SREBP1 decreased again in a concentration-dependent manner (FIGS. 9D, 9E and 9F).

Acetyl CoA carboxylase (ACC) is known to be phosphorylated by AMP-activating protein kinase (AMPK) to promote the oxidation (lipolysis) of fatty acids. Regarding the expression level of phospho-Acetyl CoA carboxylase (pACC), it was confirmed that pACC expression decreased when lipid accumulation was induced with oleic acid in HepG2 cells, and when the cells were treated with a peptide, the suppressed expression level of pACC increased again in a concentration-dependent manner (FIGS. 9G, 9H and 9I).

HSL (Hormone-sensitive lipase) is a lipid-hydrolyzing enzyme that has the activity of breaking down triacylglycerols into free fatty acids and diacylglycerols in adipose tissue, or breaking down diacylglycerols into free fatty acids and monoglycerides. HSL is activated by phosphorylation of the protein kinase PKA (Protein Kinase A), which is activated during hormone-induced signaling. Regarding the expression level of pHSL (phospho-hormone-sensitive lipase), it was confirmed that pHSL expression decreased when lipid accumulation was induced with oleic acid in HepG2 cells, and when the cells were treated with a peptide, the suppressed pHSL expression level increased again in a concentration-dependent manner (FIGS. 9J, 9K and 9L).

ATGL (Adipose triglyceride lipase) has the activity of breaking down and releasing fatty acids from triacylglycerols during the intracellular lipolysis process, enabling the released fatty acids to be used for energy. Regarding the expression level of ATGL (Adipose triglyceride lipase), it was confirmed that ATGL expression decreased when lipid accumulation was induced with oleic acid in HepG2 cells, and when the cells were treated with a peptide, the suppressed ATG expression level increased again in a concentration-dependent manner (FIGS. 9M, 9N and 9O).

Perilipin (PLIN) is one of the proteins that make up the structure of lipid droplets and is known to promote the breakdown of triacylglycerols by lipolytic enzymes. When lipolytic hormone signaling is activated, intracellular cAMP levels increase, and after the protein kinase PKA is activated, the activated PKA phosphorylates PLIN, thereby activating PLIN. Regarding the expression level of PLIN (Perilipin), it was confirmed that when lipid accumulation was induced with oleic acid in HepG2 cells, the expression of PLIN decreased, and when the cells were treated with a peptide, the suppressed expression level of PLIN increased again in a concentration-dependent manner (FIGS. 9P, 9Q and 9R).

The above experimental results demonstrate that the peptide of SEQ ID NO: 1 of the present invention has the activity of inhibiting the synthesis of lipids in hepatocytes and promoting lipolysis.

### Test Example 10: Analysis of gene expression related to insulin resistance and sensitivity in hepatocytes

The effect of the peptide of SEQ ID NO: 1 prepared in Preparation Example 1 on the expression of genes related to insulin resistance and sensitivity in hepatocytes was analyzed.

HepG2 cells (human-derived hepatocytes) were cultured in DMEM medium containing 1% P/S and 10% FBS. When cell confluency reached approximately 70 to 80%, cells were seeded into a 12-well culture plate at a concentration of 2 x 10⁵ cells/ml. Subsequently, when cell confluency in the 12-well culture plate reached approximately 70 to 80%, the medium was replaced with serum-free DMEM medium. After 4 hours, an insulin resistance environment was induced by replacing the medium with serum-free DMEM (1% BSA) supplemented with 500 µM oleic acid (Sigma, St. Louis, MO, USA). After induction for 24 hours, the cells were replaced with serum-free DMEM (1% BSA) supplemented with 500 µM oleic acid (Sigma, St. Louis, MO, USA) and peptides (5 µM, 50 µM), and cultured for 48 hours. After culture, cells were harvested and RNA was extracted using Trizol (Thermo Fisher Scientific, USA). The extracted RNA was synthesized to obtain DNA complementary to the RNA using TOPScript^{™} RT DryMIX (enzynomics, Korea), and then polymerase chain reaction (PCR) was performed on genes of markers related to insulin resistance and sensitivity using TOPsimple^{™} DryMIX-nTaq (enzynomics, Korea). Subsequently, the reaction products were run on a 1.5% agarose gel to compare the mRNA expression levels of the markers for each sample. The primer sequences for the genes used in the PCR are listed in Table 4 below, and the GAPDH gene was used as a control group.

**[Table 4]**

| Primer Name | Sequence (5'-> 3') | SEQ ID NO |
|---|---|---|
| mTOR Forward | CTG GGA CTC AAA TGT GTG CAG TTC | 20 |
| mTOR Reverse | GAA CAA TAG GGT GAA TGA TCC GGG | 21 |
| P70S6k Forward | ACT TCT GGC TCG AAA GGT GG | 22 |
| P70S6k Reverse | TTG AGT CAT CTG GGC TGT CG | 23 |
| AKT Forward | TTG TCA TGG AGT ACG CCA ACG | 24 |
| AKT Reverse | ACA GCC CGA AGT CTG TGA TCT T | 25 |
| SIRT1 Forward | TTG GCA CAG ATC CTC GAA CAA | 26 |
| SIRT1 Reverse | AAG TCT ACA GCA AGG CGA GC | 27 |
| AMPKα Forward | GTC ATG ATA GCT TGC ATA AAT GGT G | 28 |
| AMPKα Reverse | AGT TGA ATA GAA CAA GCC CTG GAC | 29 |
| GAPDH Forward | GAG TCA ACG GAT TTG GTC GT | 30 |
| GAPDH Reverse | GAC AAG CTT CCC GTT CTC AG | 31 |

Through the above experiment, it was aimed to determine whether insulin sensitivity is induced by treating HepG2 cells, in which an insulin resistance environment was induced by oleic acid treatment, with peptides. Whether insulin sensitivity was induced was confirmed by analyzing the expression levels of factors related to insulin resistance or sensitivity using RT-PCR. As a result of the experiment, it was confirmed that treatment with oleic acid increased the expression levels of mTOR and P70S6K, which are factors related to insulin resistance, in HepG2 cells, but when the cells were treated with a peptide, the increased expression levels of mTOR and P70S6K decreased again in a concentration-dependent manner of the peptide. Additionally, it was confirmed that treatment with oleic acid decreased the expression levels of AKT, SIRT1, and AMPKα, which are factors related to insulin sensitivity, in HepG2 cells, but when the cells were treated with a peptide, the decreased expression levels of AKT, SIRT1, and AMPKα increased again (FIGS. 10A and 10B).

### Test Example 11: Analysis of expression of insulin resistance and sensitivity-related proteins in hepatocytes

The effect of the peptide of SEQ ID NO: 1 prepared in Preparation Example 1 on the expression of proteins related to insulin resistance and sensitivity in hepatocytes was analyzed.

HepG2 cells (human-derived hepatocytes) were cultured in DMEM medium containing 1% P/S and 10% FBS. When cell confluency reached approximately 70 to 80%, cells were seeded into a 12-well culture plate at a concentration of 2 x 10⁵ cells/ml. Subsequently, when cell confluency in the 12-well culture plate reached approximately 70 to 80%, the medium was replaced with serum-free DMEM medium. After 4 hours, insulin resistance was induced by replacing the medium with serum-free DMEM (1% BSA) supplemented with 500 µM oleic acid (Sigma, St. Louis, MO, USA). After induction for 24 hours, the cells were replaced with serum-free DMEM (1% BSA) supplemented with 500 µM oleic acid (Sigma, St. Louis, MO, USA) and peptides (5 µM, 50 µM), and cultured for 48 hours. After culture, lysis buffer was added to lyse the cells, and the cell lysate was centrifuged at 12,000 rpm and 4°C for 30 minutes to obtain the protein. The obtained protein was quantified using a BCA kit. The protein was electrotransferred onto a membrane after performing SDS-PAGE (sodium dodecyl sulfate-polyacrylamide gel electrophoresis). After the membrane onto which the protein had been electrotransferred was blocked by treatment with 5% skim milk, it was treated with the primary antibody and incubated overnight at 4°C. The primary antibodies used are as follows: anti-pmTOR antibody (Cell signaling Technology, USA); anti-pJNK antibody (Santa Cruz, USA); anti-GLUT4 antibody (Invitrogen, USA); anti-SIRT1 antibody (Cell signaling Technology, USA); anti-pAMPKα antibody (Cell signaling Technology, USA); anti-β-actin antibody (Santa Cruz, USA). The reactant was washed with PBS-T, then reacted with secondary antibodies (Peroxidase-conjugated AffiniPure Goat Anti-Rabbit IgG (H+L), Jackson immunoResearch, USA; Peroxidase-conjugated AffiniPure Goat Anti-Mouse IgG (H+L), Jackson immunoResearch, USA) at room temperature for 1 hour, washed again with PBS-T, and then visualized by treatment with a Western detection reagent (Elpis Biotech, Daejeon, Korea) using Gel Doc (Bio-Rad, Hercules, CA, USA).

Through the above experiment, it was aimed to determine whether insulin sensitivity is induced by treating HepG2 cells, in which an insulin resistance environment was induced by oleic acid treatment, with peptides. Whether insulin sensitivity was induced was confirmed by analyzing the expression levels of factors related to insulin resistance or sensitivity using Western blot. Experimental results showed that in HepG2 cells, oleic acid treatment increased the expression levels of pmTOR and pJNK, which are factors related to insulin resistance; however, when the cells were treated with a peptide, the increased expression levels of pmTOR and pJNK decreased again. Additionally, in HepG2 cells, oleic acid treatment decreased the expression levels of pAMPKα, GLUT4, and SIRT1, which are factors related to insulin sensitivity; however, when the cells were treated with a peptide, the decreased expression levels of pAMPKα, GLUT4, and SIRT1 increased again (FIGS. 11A and 11B).

### Test Example 12: Analysis of fatty acid oxidation-related gene expression in hepatocytes

The effect of the peptide of SEQ ID NO: 1 prepared in Preparation Example 1 on the expression of fatty acid oxidation-related genes in hepatocytes (hepatocytes) was analyzed.

HepG2 cells (human-derived hepatocytes) were cultured in DMEM medium containing 1% P/S and 10% FBS. When cell confluency reached approximately 70 to 80%, cells were seeded into a 12-well culture plate at a concentration of 2 x 10⁵ cells/ml. Subsequently, when cell confluency in the 12-well culture plate reached approximately 70 to 80%, the medium was replaced with serum-free DMEM medium. After 4 hours, lipid accumulation was induced by replacing the solution with serum-free DMEM (1% BSA) supplemented with 500 µM oleic acid (Sigma, St. Louis, MO, USA). After inducing lipid accumulation for 24 hours, the solution was replaced with serum-free DMEM (1% BSA) supplemented with 500 µM oleic acid (Sigma, St. Louis, MO, USA) and peptides (5 µM, 50 µM), and cultured for 48 hours. After culture, cells were harvested and RNA was extracted using Trizol (Thermo Fisher Scientific, USA). The extracted RNA was synthesized to obtain DNA complementary to the RNA using TOPScript^{™} RT DryMIX (enzynomics, Korea), and then polymerase chain reaction (PCR) was performed on the genes of fatty acid oxidation-related markers using TOPsimple^{™} DryMIX-nTaq (enzynomics, Korea). Subsequently, the reaction products were run on a 1.5% agarose gel to compare the mRNA expression levels of the aforementioned markers for each sample. The primer sequences for the genes used in the PCR are listed in Table 5 below, and the GAPDH gene was used as a control.

**[Table 5]**

| Primer Name | Sequence (5'-> 3') | SEQ ID NO |
|---|---|---|
| CPT1 Forward | CCT CCA GTT GGC TTA TCG TG | 32 |
| CPT1 Reverse | TTC TTC GTC TGG CTG GAC AT | 33 |
| PGC1α Forward | AGT CTG TAT GGA GTG ACA TCG AG | 34 |
| PGC1α Reverse | GGC AAT CCG TCT TCA TCC AC | 35 |
| PPARα Forward | AAG GGC TTC TTT CGG CGA AC | 36 |
| PPARα Reverse | TGA CCT TGT TCA TGT TGA AGT TCT TCA | 37 |
| GAPDH Forward | GAG TCA ACG GAT TTG GTC GT | 30 |
| GAPDH Reverse | GAC AAG CTT CCC GTT CTC AG | 31 |

As a result of the experiment, treatment with oleic acid in HepG2 cells reduced the expression of factors related to fatty acid oxidation, namely CPT1 (Carnitine Palmitoyltransferase I), PGC1α (Peroxisome proliferator-activated receptor gamma coactivator 1-alpha), and PPARα (Peroxisome Proliferator-Activated Receptor α). When the cells were treated with a peptide, the reduced expression levels of CPT1, PGC1α, and PPARα increased again (FIGS. 12A and 12B).

### Test Example 13: Analysis of fatty acid oxidation-related protein expression in hepatocytes

The effect of the peptide of SEQ ID NO: 1 prepared in Preparation Example 1 on the expression of fatty acid oxidation-related proteins in hepatocytes (hepatocytes) was analyzed.

HepG2 cells (human-derived hepatocytes) were cultured in DMEM medium containing 1% P/S and 10% FBS. When cell confluency reached approximately 70 to 80%, cells were seeded into a 12-well culture plate at a concentration of 2 x 10⁵ cells/ml. Subsequently, when cell confluency in the 12-well culture plate reached approximately 70 to 80%, the medium was replaced with serum-free DMEM medium. After 4 hours, lipid accumulation was induced by replacing the solution with serum-free DMEM (1% BSA) supplemented with 500 µM oleic acid (Sigma, St. Louis, MO, USA). After inducing lipid accumulation for 24 hours, the solution was replaced with serum-free DMEM (1% BSA) supplemented with 500 µM oleic acid (Sigma, St. Louis, MO, USA) and peptides (5 µM, 50 µM), and cultured for 48 hours. After culture, lysis buffer was added to lyse the cells, and the cell lysate was centrifuged at 4°C at 12,000 rpm for 30 minutes to obtain the protein. The obtained protein was quantified using a BCA kit. The protein was electrotransferred onto a membrane after performing SDS-PAGE (sodium dodecyl sulfate-polyacrylamide gel electrophoresis). After blocking the protein-attached membrane with 5% skim milk, the membrane was treated with the primary antibody and incubated overnight at 4°C. The primary antibodies used are as follows: anti-CPT1 antibody (Abcam, UK), anti-PGC1α antibody (Abcam, UK), and anti-β-actin antibody (Santa Cruz, USA). The reaction mixture was washed with PBS-T, then reacted with secondary antibodies (Peroxidase-conjugated AffiniPure Goat Anti-Rabbit IgG (H+L), Jackson immunoResearch, USA; Peroxidase-conjugated AffiniPure Goat Anti-Mouse IgG (H+L), Jackson immunoResearch, USA) at room temperature for 1 hour, washed again with PBS-T, and then visualized by treatment with a Western detection reagent (Elpis Biotech, Daejeon, Korea) using Gel Doc (Bio-Rad, Hercules, CA, USA).

Experimental results showed that the expression of CPT1 (Carnitine Palmitoyltransferase I) and PGC1α (Peroxisome proliferator-activated receptor gamma coactivator 1-alpha) proteins, which are factors related to fatty acid oxidation, decreased in HepG2 cells due to oleic acid treatment; however, when a peptide was applied, the reduced expression levels of CPT1 and PGC1α proteins increased again (FIG. 13).

### [Test Examples 14 to 22] Test Example for adipocytes

### Test Example 14: Analysis of insulin resistance and sensitivity-related protein expression in adipocytes

The effect of the peptide of SEQ ID NO: 1 prepared in Preparation Example 1 on the expression of proteins related to insulin resistance and sensitivity in adipocytes was analyzed.

3T3-L1 cells (preadipocytes, American Type Culture Collection) were cultured in DMEM medium containing 2% BCS (Bovine Calf Serum) (Welgene, Korea). To differentiate 3T3-L1 cells into adipocytes, they were seeded into 12-well culture plates at a concentration of 1 x 10⁵ cells/well, and when they became confluent (Day=2), the medium was changed once more and cultured for an additional 48 hours. After 48 hours (Day=0), the culture medium was replaced with DMEM medium containing 10% FBS, 1% P/S, 1 µg/ml insulin (Sigma, USA), 0.5 mM isobutylmethylxanthine (IBMX) (Sigma, USA) and 1 µM Dexamethasone (Sigma, USA) and cultured. After 2 days (Day=2), the medium was replaced with DMEM containing 10% FBS, 1% P/S, and 1 µg/ml insulin, and cultured. After 3 days (Day=5), the medium was replaced with one containing only 10% FBS and 1% P/S, and peptides (5 µM, 50 µM) were added and cultured for 48 hours. After culture, lysis buffer was added to lyse the cells, and the protein was obtained by centrifugation at 4°C and 12,000 rpm for 30 minutes. The obtained protein was then quantified using a BCA kit. Subsequently, the protein was electrotransferred onto a membrane after performing SDS-PAGE (sodium dodecyl sulfate-polyacrylamide gel electrophoresis). The membrane to which the protein was attached was blocked by treatment with 5% skim milk, and then the primary antibody was incubated overnight at 4°C. The primary antibodies used are as follows: anti-pIRS (Tyr612) antibody (Invitrogen, USA); anti-GLUT4 antibody (Invitrogen, USA); anti-pIRS (Ser302) antibody (Cell signaling Technology, USA); anti-P70S6K antibody (Cell signaling Technology, USA); anti-β-actin antibody (Santa Cruz, USA). The reaction mixture was washed with PBS-T, then reacted with secondary antibodies (Peroxidase-conjugated AffiniPure Goat Anti-Rabbit IgG (H+L), Jackson immunoResearch, USA; Peroxidase-conjugated AffiniPure Goat Anti-Mouse IgG (H+L), Jackson immunoResearch, USA) at room temperature for 1 hour, washed again with PBS-T, and then visualized by treatment with a Western detection reagent (Elpis Biotech, Daejeon, Korea) using Gel Doc (Bio-Rad, Hercules, CA, USA).

In the above experiment, differentiation was induced in 3T3-L1 preadipocytes with an MDI cocktail for 5 days, and then the insulin sensitivity-promoting effect was confirmed by measuring the expression levels of insulin sensitivity or resistance proteins upon peptide treatment. As a result of the experiment, peptide treatment in 3T3-L1 cells increased the expression levels of pIRS(Tyr612) and GLUT4, which are factors related to insulin sensitivity, while decreasing the expression levels of pIRS(Ser302) and p70S6K, which are factors related to insulin resistance (FIGS. 14A and 14B).

### Test Example 15: Analysis of expression of genes related to insulin resistance and sensitivity in adipocytes

The effect of the peptide of SEQ ID NO: 1 prepared in Preparation Example 1 on the expression of genes related to insulin resistance and sensitivity in adipocytes was analyzed.

3T3-L1 cells (preadipocytes, American Type Culture Collection) were cultured in DMEM medium containing 2% BCS (Bovine Calf Serum) (Welgene, Korea). To differentiate 3T3-L1 cells into adipocytes, they were seeded into 12-well culture plates at a concentration of 1 x 10⁵ cells/well, and when they became confluent (Day=2), the medium was changed once more and cultured for an additional 48 hours. After 48 hours (Day=0), the culture medium was replaced with DMEM medium containing 10% FBS, 1% P/S, 1 µg/ml insulin (Sigma, USA), 0.5 mM isobutylmethylxanthine (IBMX) (Sigma, USA) and 1 µM Dexamethasone (Sigma, USA) and cultured. After 2 days (Day=2), the medium was replaced with DMEM containing 10% FBS, 1% P/S, and 1 µg/ml insulin, and cultured. After 3 days (Day=5), the medium was replaced with one containing only 10% FBS and 1% P/S, and an insulin resistance environment was induced by adding 350 µM oleic acid (Sigma, St. Louis, MO, USA) for 24 hours. After 24 hours of induction, the medium was replaced with DMEM (10% FBS, 1% BSA) supplemented with 350 µM oleic acid (Sigma, St. Louis, MO, USA) and peptides (5 µM, 50 µM), and cultured for 48 hours. After culture, cells were harvested, and RNA was extracted using Trizol (Thermo Fisher Scientific, USA). The extracted RNA was synthesized to obtain DNA complementary to the RNA using TOPScript^{™} RT DryMIX (enzynomics, Korea), and then polymerase chain reaction (PCR) was performed on genes of markers related to insulin resistance or sensitivity using TOPsimple^{™} DryMIX-nTaq (enzynomics, Korea). Subsequently, the reaction products were run on a 1.5% agarose gel to compare the mRNA expression levels of the aforementioned markers for each sample. The primer sequences for the genes used in the PCR are listed in Table 6 below, and the GAPDH gene was used as a control.

**[Table 6]**

| Primer Name | Sequence (5'-> 3') | SEQ ID NO |
|---|---|---|
| P70S6K Forward | GGA GCC TGG GAG CCC TGA TGT | 38 |
| P70S6K Reverse | GAA GCC CTC TTT GAT GCT GTC C | 39 |
| GLUT4 Forward | ACT AAG AGC ACC GAG ACC AA | 40 |
| GLUT4 Reverse | CTG CCC GAA AGA GTC TAA AG | 41 |
| SIRT1 Forward | GAT CCT TTG GAT TCC TGC AA | 42 |
| SIRT1 Reverse | AGT TCC AGC CGT CTC TGT GT | 43 |
| AMPKα Forward | TCA CCG GAC ATA AAG TGG CT | 44 |
| AMPKα Reverse | TGA TGA TGT GAG GGT GCC TG | 45 |
| GAPDH Forward | GTG ATG GCA TGG ACT GTG GT | 8 |
| GAPDH Reverse | GGA GCC AAA AGG GTC ATC AT | 9 |

Through the above experiment, it was aimed to determine whether insulin resistance is inhibited and insulin sensitivity is induced in 3T3-L1 adipocytes in which an insulin resistance environment was induced by oleic acid treatment by treating them with a peptide. The expression levels of relevant factors regarding the inhibition of insulin resistance or the induction of insulin sensitivity were confirmed via Western blot. As a result of the experiment, treatment with oleic acid in 3T3-L1 cells increased the expression level of p70S6K, which is an insulin resistance-related factor, and when the cells were treated with a peptide, the increased expression level of p70S6K decreased again. In addition, it was confirmed that treatment with oleic acid in 3T3-L1 cells decreased the expression levels of AMPKα, GLUT4, and SIRT1, which are insulin sensitivity-related factors, and when the cells were treated with a peptide, the decreased expression levels of AMPKα, GLUT4, and SIRT1 increased again (FIGS. 15A and 15B).

### Test Example 16: Analysis of insulin resistance and sensitivity-related protein expression in adipocytes

The effect of the peptide of SEQ ID NO: 1 prepared in Preparation Example 1 on the expression of proteins related to insulin resistance and sensitivity in adipocytes was analyzed.

3T3-L1 cells (preadipocytes, American Type Culture Collection) were cultured in DMEM medium comprising 2% BCS (Bovine Calf Serum) (Welgene, Korea). To differentiate 3T3-L1 cells into adipocytes, cells were seeded into 12-well culture plates at a concentration of 1 x 10⁵ cells/well, and when the cells became confluent (Day=2), the medium was replaced once more and cultured for an additional 48 hours. After 48 hours (Day=0), the culture medium was replaced with DMEM medium comprising 10% FBS, 1% P/S, 1 µg/ml insulin (Sigma, USA), 0.5 mM isobutylmethylxanthine (IBMX) (Sigma, USA) and 1 µM Dexamethasone (Sigma, USA) and cultured. After 2 days (Day=2), the medium was replaced with DMEM comprising 10% FBS, 1% P/S, and 1 µg/ml insulin, and cultured. After 3 days (Day=5), the medium was replaced with one comprising only 10% FBS and 1% P/S, and an insulin resistance environment was induced by adding 350 µM oleic acid (Sigma, St. Louis, MO, USA) for 24 hours. After 24 hours of induction, the medium was replaced with DMEM medium (10% FBS, 1% BSA) supplemented with 350 µM oleic acid (Sigma, St. Louis, MO, USA) and peptides (5 µM, 50 µM), and cultured for 48 hours. After culture, lysis buffer was added to lyse the cells, and then the proteins were obtained by centrifugation at 12,000 rpm and 4°C for 30 minutes. The obtained proteins were then quantified using a BCA kit. Subsequently, the protein was electrotransferred onto a membrane after performing SDS-PAGE (sodium dodecyl sulfate-polyacrylamide gel electrophoresis). The membrane to which the protein was attached was blocked by treatment with 5% skim milk, and then the primary antibody was incubated overnight at 4°C. The primary antibodies used are as follows: anti-pAMPKα antibody (Cell signaling Technology, USA), anti-pIRS (Ser302) antibody (Cell signaling Technology, USA), and anti-β-actin antibody (Santa Cruz, USA). The reactant was washed with PBS-T, then reacted with secondary antibodies (Peroxidase-conjugated AffiniPure Goat Anti-Rabbit IgG (H+L), Jackson immunoResearch, USA; Peroxidase-conjugated AffiniPure Goat Anti-Mouse IgG (H+L), Jackson immunoResearch, USA) at room temperature for 1 hour, washed again with PBS-T, and then visualized by treatment with a Western detection reagent (Elpis Biotech, Daejeon, Korea) using Gel Doc (Bio-Rad, Hercules, CA, USA).

As a result of the experiment, treatment with oleic acid in 3T3-L1 cells reduced the expression level of pAMPKα, which is a factor related to insulin sensitivity, and when the peptide was added, the reduced expression level of pAMPKα increased again. In addition, treatment with oleic acid in 3T3-L1 cells increased the expression level of pIRS(Ser302), which is a factor related to insulin resistance, and when the peptide was added, the increased expression level of pIRS(Ser302) decreased again (FIG. 16).

### Test Example 17: Analysis of adipogenesis-related gene expression in adipocytes

The effect of the peptide of SEQ ID NO: 1 prepared in Preparation Example 1 on the expression of adipogenesis-related genes in adipocytes was analyzed.

3T3-L1 cells (preadipocytes, American Type Culture Collection) were cultured in DMEM medium containing 2% BCS (Bovine Calf Serum) (Welgene, Korea). To differentiate 3T3-L1 cells into adipocytes, cells were seeded into 12-well culture plates at a concentration of 1 x 10⁵ cells/well, and when the cells became confluent (Day=2), the medium was replaced once more and cultured for an additional 48 hours. After 48 hours (Day=0), the culture medium was replaced with DMEM medium containing 10% FBS, 1% P/S, 1 µg/ml insulin (Sigma, USA), 0.5 mM isobutylmethylxanthine (IBMX) (Sigma, USA), and 1 µM Dexamethasone (Sigma, USA), and cultured. After 2 days (Day=2), the medium was replaced with DMEM medium containing 10% FBS, 1% P/S, and 1 µg/ml insulin, and cultured. After 3 days (Day=5), the medium was replaced with one containing only 10% FBS and 1% P/S, treated with peptides (5 µM, 50 µM), and cultured for 48 hours. After culture, cells were harvested and RNA was extracted using Trizol (Thermo Fisher Scientific, USA). DNA complementary to the extracted RNA was synthesized using TOPScript^{™} RT DryMIX (enzynomics, Korea), and then polymerase chain reaction (PCR) was performed on genes of adipogenesis-related markers using TOPsimple^{™} DryMIX-nTaq (enzynomics, Korea). Subsequently, the reaction products were run on a 1.5% agarose gel to compare the mRNA expression levels of the markers for each sample. The primer sequences for the genes used in the PCR are listed in Table 7 below, and the GAPDH gene was used as a control group.

**[Table 7]**

| Primer Name | Sequence (5'-> 3') | SEQ ID NO |
|---|---|---|
| ACCα Forward | ACC TTA CTG CCA TCC CAT GTG CTA | 46 |
| ACCα Reverse | GTG CCT GAT GAT CGC ACG AAC AAA | 47 |
| PPARγ Forward | TCC GCT GAT GCA CTG CCT AT | 48 |
| PPARγ Reverse | ACA GAC TCG GCA CTC AAT GG | 49 |
| GAPDH Forward | GTG ATG GCA TGG ACT GTG GT | 8 |
| GAPDH Reverse | GGA GCC AAA AGG GTC ATC AT | 9 |

ACCα (Acetyl-CoA Carboxylase-alpha) is a gene for an enzyme involved in adipogenesis, catalyzing the carboxylation of acetyl-CoA to malonyl-CoA, which is the rate-limiting step in fatty acid synthesis. PPAR-γ (Peroxisome proliferator-activated receptor gamma) is known to regulate fatty acid storage and glucose metabolism, and is known to increase the ability of adipocytes to store fatty acids. Experimental results confirmed that treatment of 3T3-L1 cells with the peptide reduced the expression of ACCα and PPAR-γ (FIG. 17).

### Test Example 18 : Analysis of adipogenesis-related protein expression in adipocytes

The effect of the peptide of SEQ ID NO: 1 prepared in Preparation Example 1 on the expression of adipogenesis-related proteins in adipocytes was analyzed.

3T3-L1 cells (preadipocytes, American Type Culture Collection) were cultured in DMEM medium containing 2% BCS (Bovine Calf Serum) (Welgene, Korea). To differentiate 3T3-L1 cells into adipocytes, cells were seeded into 12-well culture plates at a concentration of 1 x 10⁵ cells/well, and when the cells became confluent (Day=2), the medium was replaced once more and cultured for an additional 48 hours. After 48 hours (Day=0), the culture medium was replaced with DMEM medium containing 10% FBS, 1% P/S, 1 µg/ml insulin (Sigma, USA), 0.5 mM isobutylmethylxanthine (IBMX) (Sigma, USA), and 1 µM Dexamethasone (Sigma, USA), and cultured. After 2 days (Day=2), the medium was replaced with DMEM medium containing 10% FBS, 1% P/S, and 1 µg/ml insulin, and cultured. After 3 days (Day=5), the medium was replaced with one containing only 10% FBS and 1% P/S, treated with peptides (5 µM, 50 µM), and cultured for 48 hours. After culturing, lysis buffer was added to lyse the cells, and then the protein was obtained by centrifuging at 12,000 rpm and 4°C for 30 minutes. The obtained protein was then quantified using a BCA kit. Subsequently, the protein was electrotransferred onto a membrane after performing SDS-PAGE (sodium dodecyl sulfate-polyacrylamide gel electrophoresis). The membrane to which the protein was attached was blocked by treatment with 5% skim milk, and then the primary antibody was incubated overnight at 4°C. The primary antibodies used are as follows: anti-SREBP1 antibody (Abcam, US); anti-FAS antibody (Cell signaling Technology, USA); anti-C/EBPα antibody (Cell signaling Technology, USA); anti-pACCα antibody (Cell signaling Technology, USA); anti-β-actin antibody (Santa Cruz, USA). The reactant was washed with PBS-T, then reacted with secondary antibodies (Peroxidase-conjugated AffiniPure Goat Anti-Rabbit IgG (H+L), Jackson immunoResearch, USA; Peroxidase-conjugated AffiniPure Goat Anti-Mouse IgG (H+L), Jackson immunoResearch, USA) at room temperature for 1 hour, washed again with PBS-T, and then visualized by treatment with a Western detection reagent (Elpis Biotech, Daejeon, Korea) using Gel Doc (Bio-Rad, Hercules, CA, USA).

In the above experiment, 3T3-L1 adipocytes were differentiated using an MDI cocktail, and then the inhibitory effect on adipogenesis caused by peptide treatment was confirmed by measuring the expression levels of related factors. SREBP1 (Sterol Regulatory Element Binding Protein-1) is known to induce the synthesis of lipids and cholesterol, FAS is a fatty acid synthase, and C/EBPα (CCAAT/enhancer-binding protein α) is known to induce adipogenesis through PPAR-γ (Genes Dev. 2002 Jan 1; 16(1): 22-26). In addition, ACCα (Acetyl CoA carboxylase α) is known to be phosphorylated by AMPK (AMP-activating protein kinase) to promote the oxidation (degradation) of fatty acids. Experimental results showed that in 3T3-L1 adipocytes, peptide treatment reduced the expression levels of adipogenesis-related factors SREBP1, FAS, and C/EBPα, while conversely, the expression level of pACCα, which promotes the degradation of fatty acids, increased (FIGS. 18A and 18B).

### Test Example 19 : Analysis of lipolysis-related protein expression in adipocytes

The effect of the peptide of SEQ ID NO: 1 prepared in Preparation Example 1 on the expression of lipolysis-related proteins in adipocytes was analyzed. 3T3-L1 cells (preadipocytes, American Type Culture Collection) were cultured in DMEM medium containing 2% BCS (Bovine Calf Serum) (Welgene, Korea). To differentiate 3T3-L1 cells into adipocytes, cells were seeded into 12-well culture plates at a concentration of 1 x 10⁵ cells/well, and when the cells became confluent (Day=2), the medium was replaced once more and cultured for an additional 48 hours. After 48 hours (Day=0), the culture medium was replaced with DMEM medium containing 10% FBS, 1% P/S, 1 µg/ml insulin (Sigma, USA), 0.5 mM isobutylmethylxanthine (IBMX) (Sigma, USA), and 1 µM Dexamethasone (Sigma, USA), and cultured. After 2 days (Day=2), the medium was replaced with DMEM medium containing 10% FBS, 1% P/S, and 1 µg/ml insulin, and cultured. After 3 days (Day=5), the medium was replaced with one containing only 10% FBS and 1% P/S, treated with peptides (5 µM, 50 µM), and cultured for 48 hours. After culturing, lysis buffer was added to lyse the cells, and the protein was obtained by centrifuging at 12,000 rpm and 4°C for 30 minutes. The obtained protein was then quantified using a BCA kit. Subsequently, the protein was electrotransferred onto a membrane after performing SDS-PAGE (sodium dodecyl sulfate-polyacrylamide gel electrophoresis). The membrane to which the protein was attached was blocked by treatment with 5% skim milk, and then the primary antibody was incubated overnight at 4°C. The primary antibodies used are as follows: anti-PLIN antibody (Abcam, US); anti-pHSL antibody (Cell signaling Technology, USA); anti-PGC1α antibody (Cell signaling Technology, USA); anti-β-actin antibody (Santa Cruz, USA). The reaction mixture was washed with PBS-T, then reacted with secondary antibodies (Peroxidase-conjugated AffiniPure Goat Anti-Rabbit IgG (H+L), Jackson immunoResearch, USA; Peroxidase-conjugated AffiniPure Goat Anti-Mouse IgG (H+L), Jackson immunoResearch, USA; Peroxidase-conjugated AffiniPure Rabbit Anti-Goat IgG (H+L), Jackson immunoResearch, USA) at room temperature for 1 hour, washed again with PBS-T, and then visualized using Gel Doc (Bio-Rad, Hercules, CA, USA) by treatment with a Western detection reagent (Elpis Biotech, Daejeon, Korea).

Through the above experiment, 3T3-L1 adipocytes were induced to differentiate using an MDI cocktail, and then the promoting effect of lipolysis by peptide treatment was confirmed by measuring the expression levels of related factors. PLIN (Perilipin) is phosphorylated by activated PKA and promotes the degradation of triacylglycerols by lipases. HSL (Hormone-sensitive lipase) is an enzyme that hydrolyzes fat and is activated by phosphorylation by the activated kinase PKA (Protein Kinase A). PGC1α (Peroxisome proliferator-activated receptor gamma coactivator 1-alpha) is a transcriptional coactivator that regulates the expression of genes related to energy metabolism (Adv Physiol Educ. 2006 Dec;30(4):145-51). Experimental results confirmed that when 3T3-L1 adipocytes were treated with the peptide, the expression levels of PLIN, pHSL, and PGC1α, which are factors related to lipolysis, increased (FIG. 19).

### Test Example 20: Analysis of fatty acid oxidation-related gene expression after induction of insulin resistance environment in adipocytes

The effect of the peptide of SEQ ID NO: 1 prepared in Preparation Example 1 on the expression of genes related to fatty acid oxidation in insulin resistance-induced adipocytes was analyzed.

3T3-L1 cells (preadipocytes, American Type Culture Collection) were cultured in DMEM medium containing 2% BCS (Bovine Calf Serum) (Welgene, Korea). To differentiate 3T3-L1 cells into adipocytes, cells were seeded into 12-well culture plates at a concentration of 1 x 10⁵ cells/well, and when the cells became confluent (Day=2), the medium was replaced once more and cultured for an additional 48 hours. After 48 hours (Day=0), the culture medium was replaced with DMEM medium containing 10% FBS, 1% P/S, 1 µg/ml insulin (Sigma, USA), 0.5 mM isobutylmethylxanthine (IBMX) (Sigma, USA), and 1 µM Dexamethasone (Sigma, USA), and cultured. After 2 days (Day=2), the medium was replaced with DMEM medium containing 10% FBS, 1% P/S, and 1 µg/ml insulin, and cultured. After 3 days (Day=5), the medium was replaced with one containing only 10% FBS and 1% P/S, and an insulin resistance environment was induced by treating with 350 µM oleic acid (Sigma, St. Louis, MO, USA) for 24 hours. After 24 hours of induction, the DMEM medium (10% FBS, 1% BSA) supplemented with 350 µM oleic acid (Sigma, St. Louis, MO, USA) and peptides (5 µM, 50 µM) was replaced, and the cells were cultured for 48 hours. After culture, the cells were harvested, and RNA was extracted using Trizol (Thermo Fisher Scientific, USA). The extracted RNA was synthesized to obtain DNA complementary to the RNA using TOPScript^{™} RT DryMIX (enzynomics, Korea), and then polymerase chain reaction (PCR) was performed on the genes of fatty acid oxidation-related markers using TOPsimple^{™} DryMIX-nTaq (enzynomics, Korea). Subsequently, the reaction products were run on a 1.5% agarose gel to compare the mRNA expression levels of the markers for each sample. The primer sequences for the genes used in the PCR are listed in Table 8 below, and the GAPDH gene was used as a control group.

**[Table 8]**

| Primer Name | Sequence (5'-> 3') | SEQ ID NO |
|---|---|---|
| PGC1α Forward | ATG TGC AGC CAA GAC TCT GTA | 50 |
| PGC1α Reverse | CGC TAC ACC ACT TCA ATC CAC | 51 |
| CPT1 Forward | CGT ACC AAG TAG CCA AGG CA | 52 |
| CPT1 Reverse | CAG GAA CGC ACA GTC TCA GT | 53 |
| GAPDH Forward | GTG ATG GCA TGG ACT GTG GT | 8 |
| GAPDH Reverse | GGA GCC AAA AGG GTC ATC AT | 9 |

As a result of the experiment, the expression of PGC1α (Peroxisome proliferator-activated receptor gamma coactivator 1-alpha) and CPT1 (Carnitine Palmitoyltransferase I), which are genes related to fatty acid oxidation, decreased in 3T3-L1 cells due to oleic acid treatment, but when the cells were treated with a peptide, the decreased expression of PGC1α and CPT1 increased again (FIG. 20).

### Test Example 21: Analysis of gene expression related to adipogenesis and lipolysis after inducing an insulin resistance environment in adipocytes

The effect of the peptide of SEQ ID NO: 1 prepared in Preparation Example 1 on the expression of genes related to adipogenesis and lipolysis in insulin resistance-induced adipocytes was analyzed.

3T3-L1 cells (preadipocytes, American Type Culture Collection) were cultured in DMEM medium containing 2% BCS (Bovine Calf Serum) (Welgene, Korea). To differentiate 3T3-L1 cells into adipocytes, cells were seeded into 12-well culture plates at a concentration of 1 x 10⁵ cells/well, and when the cells became confluent (Day=2), the medium was replaced once more and cultured for an additional 48 hours. After 48 hours (Day=0), the culture medium was replaced with DMEM medium containing 10% FBS, 1% P/S, 1 µg/ml insulin (Sigma, USA), 0.5 mM isobutylmethylxanthine (IBMX) (Sigma, USA), and 1 µM Dexamethasone (Sigma, USA), and cultured. After 2 days (Day=2), the medium was replaced with DMEM medium containing 10% FBS, 1% P/S, and 1 µg/ml insulin, and cultured. After 3 days (Day=5), the medium was replaced with one containing only 10% FBS and 1% P/S, and an insulin resistance environment was induced by treating with 350 µM oleic acid (Sigma, St. Louis, MO, USA) for 24 hours. After 24 hours of induction, the DMEM medium (10% FBS, 1% BSA) supplemented with 350 µM oleic acid (Sigma, St. Louis, MO, USA) and peptides (5 µM, 50 µM) was replaced, and the cells were cultured for 48 hours. After culture, the cells were harvested, and RNA was extracted using Trizol (Thermo Fisher Scientific, USA). The extracted RNA was used to synthesize DNA complementary to the RNA using TOPScript^{™} RT DryMIX (enzynomics, Korea), and then polymerase chain reaction (PCR) was performed on genes of markers related to adipogenesis and lipolysis using TOPsimple^{™} DryMIX-nTaq (enzynomics, Korea). Subsequently, the reaction products were run on a 1.5% agarose gel to compare the mRNA expression levels of the markers for each sample. The primer sequences for the genes used in the PCR are listed in Table 9 below, and the GAPDH gene was used as a control group.

**[Table 9]**

| Primer Name | Sequence (5'-> 3') | SEQ ID NO |
|---|---|---|
| FAS Forward | TGC TGG CAC TAC AGA ATG C | 54 |
| FAS Reverse | AAC AGC CTC AGA GCG ACA AT | 55 |
| HSL Forward | GGA CAC ACA CAC ACC TG | 56 |
| HSL Reverse | CCC TTT CGC AGC AAC TTT AG | 57 |
| ATGL Forward | TCG TGG ATG TTG GTG GAG CT | 58 |
| ATGL Reverse | TGT GGC CTC ATT CCT CCT A | 59 |
| PLIN Forward | AAG GAT CCT GCA CCT CAC AC | 60 |
| PLIN Reverse | CCT CTG CTG AAG GGT TAT CG | 61 |
| GAPDH Forward | GTG ATG GCA TGG ACT GTG GT | 8 |
| GAPDH Reverse | GGA GCC AAA AGG GTC ATC AT | 9 |

Through the above experiment, after inducing an insulin resistance environment in 3T3-L1 adipocytes, the inhibitory effect on adipogenesis or the promotion effect of lipolysis by peptide treatment was confirmed by measuring the expression levels of related factors. Experimental results showed that in 3T3-L1 adipocytes, treatment with oleic acid increased the expression level of the FAS gene, which is a factor related to adipogenesis; however, when the peptide was added, the increased expression level of the FAS gene decreased again. Additionally, in 3T3-L1 adipocytes, treatment with oleic acid decreased the expression levels of the PLIN, HSL, and ATGL genes, which are factors related to lipolysis; however, when the peptide was added, the decreased expression levels of the PLIN, HSL, and ATGL genes increased again (FIGS. 21A and 21B).

### Test Example 22: Analysis of expression of proteins related to adipogenesis and lipolysis after induction of insulin resistance environment in adipocytes

The effect of the peptide of SEQ ID NO: 1 prepared in Preparation Example 1 on the expression of proteins related to adipogenesis and lipolysis in insulin resistance-induced adipocytes was analyzed.

3T3-L1 cells (preadipocytes, American Type Culture Collection) were cultured in DMEM medium containing 2% BCS (Bovine Calf Serum) (Welgene, Korea). To differentiate 3T3-L1 cells into adipocytes, cells were seeded into 12-well culture plates at a concentration of 1 x 10⁵ cells/well, and when the cells became confluent (Day=2), the medium was replaced once more and cultured for an additional 48 hours. After 48 hours (Day=0), the culture medium was replaced with DMEM medium containing 10% FBS, 1% P/S, 1 µg/ml insulin (Sigma, USA), 0.5 mM isobutylmethylxanthine (IBMX) (Sigma, USA), and 1 µM Dexamethasone (Sigma, USA), and cultured. After 2 days (Day=2), the medium was replaced with DMEM medium containing 10% FBS, 1% P/S, and 1 µg/ml insulin, and cultured. After 3 days (Day=5), the medium was replaced with one containing only 10% FBS and 1% P/S, and an insulin resistance environment was induced by treating with 350 µM oleic acid (Sigma, St. Louis, MO, USA) for 24 hours. After 24 hours of induction, the DMEM medium (10% FBS, 1% BSA) supplemented with 350 µM oleic acid (Sigma, St. Louis, MO, USA) and peptides (5 µM, 50 µM) were replaced, and the cells were cultured for 48 hours. After culture, lysis buffer was added to lyse the cells, and the proteins were obtained by centrifugation at 12,000 rpm and 4°C for 30 minutes. The obtained proteins were then quantified using a BCA kit. Subsequently, the protein was electrotransferred onto a membrane after performing SDS-PAGE (sodium dodecyl sulfate-polyacrylamide gel electrophoresis). The membrane to which the protein was attached was blocked by treatment with 5% skim milk, and then the primary antibody was incubated overnight at 4°C. The primary antibodies used are as follows: anti-SREBP1 antibody (Abcam, US); anti-FAS antibody (Cell signaling Technology, USA); anti-PLIN antibody (Abcam, US); anti-β-actin antibody (Santa Cruz, USA). The reactant was washed with PBS-T, then reacted with secondary antibodies (Peroxidase-conjugated AffiniPure Goat Anti-Rabbit IgG (H+L), Jackson immunoResearch, USA; Peroxidase-conjugated AffiniPure Goat Anti-Mouse IgG (H+L), Jackson immunoResearch, USA; Peroxidase-conjugated AffiniPure Rabbit Anti-Goat IgG (H+L), Jackson immunoResearch, USA) at room temperature for 1 hour, washed again with PBS-T, and then visualized using Gel Doc (Bio-Rad, Hercules, CA, USA) by treatment with a Western detection reagent (Elpis Biotech, Daejeon, Korea).

Experimental results showed that in 3T3-L1 adipocytes, treatment with oleic acid increased the expression level of FAS protein, which is a factor related to adipogenesis; however, when peptides were added, the increased expression level of FAS protein decreased again. Additionally, in 3T3-L1 adipocytes, treatment with oleic acid decreased the expression levels of SREBP1 and PLIN proteins, which are factors related to lipolysis; however, when peptides were added, the decreased expression levels of SREBP1 and PLIN proteins increased again (FIGS. 22A and 22B).

Although representative embodiments of the present application have been described above by way of example, the scope of the present application is not limited to such specific embodiments, and those skilled in the art will be able to make appropriate modifications within the scope described in the claims of the present application.

## Claims

1. A peptide comprising the amino acid sequence of SEQ ID NO: 1.

2. The peptide of claim 1, wherein the peptide has the following activities:
(i) activity that promotes the gene expression of cell proliferation-related factor PCNA (Proliferating cell nuclear antigen), NDRG2 (N-myc Downstream-Regulated Gene 2), or CDK4 (Cyclin-dependent kinase 4) in myoblasts;
(ii) activity that promotes the protein expression of cell proliferation-related factor SIRT1 (Sirtuin 1), Ki67, Pax7, or pAKT in myoblasts;
(iii) activity that promotes the gene expression of differentiation-related factor MyoD, Myf6, mTOR, Myf5, or Myf4 in myoblasts;
(iv) activity that promotes the protein expression of differentiation-related factor α-actinin, Myf6, or MyoG in myoblasts; or
(v) activity that promotes the protein expression of signaling factor involved in muscle protein synthesis, SIRT1, pAMPKα, pAKT, pmTOR, or p70S6K in myoblasts.

3. The peptide of claim 1, wherein the peptide has the following activities:
(i) activity that inhibits lipid accumulation in hepatocytes;
(ii) activity that inhibits the protein expression of adipogenesis factor SREBP1 or FAS and promotes the protein expression of lipolysis factor pACC, pHSL, ATGL, or PLIN in hepatocytes where lipid accumulation is induced;
(iii) activity that promotes the gene expression of fatty acid oxidation-related factor CPT1, PGC1α, or PPARα and promotes the protein expression of fatty acid oxidation-related factor CPT1 or PGC1α in hepatocytes where lipid accumulation is induced;
(iv) activity that inhibits the gene expression of adipogenesis-related factor ACCα or PPAR-γ in adipocytes;
(v) activity that inhibits the protein expression of adipogenesis-related factor SREBP1, FAS, or C/EBPα and promotes the protein expression of fatty acid degradation-related factor pACCα in adipocytes;
(vi) activity that promotes the protein expression of lipolysis-related factor PLIN, pHSL, or PGC1α in adipocytes;
(vii) activity that promotes the gene expression of fatty acid oxidation-related factor PGC1α or CPT1 in adipocytes where insulin resistance is induced;
(viii) activity that inhibits the gene expression of fatty acid synthesis factor FAS and promotes the gene expression of lipolysis-related factor PLIN, HSL, or ATGL in adipocytes where insulin resistance is induced; or
(ix) activity that inhibits the protein expression of fatty acid synthesis factor FAS or SREBP1 and promotes the protein expression of lipolysis-related factor PLIN in adipocytes where insulin resistance is induced.

4. The peptide of claim 1, wherein the peptide has the following activities:
(i) activity that promotes the protein expression of insulin sensitivity factor pIRS (Tyr612) or GLUT4 and inhibits the protein expression of insulin resistance factor pIRS (Ser302) or p70S6K in adipocytes;
(ii) activity that inhibits the gene expression of the insulin resistance factor P70S6K and promotes the gene expression of the insulin sensitivity factor AMPKα, GLUT4, or SIRT1 in adipocytes where insulin resistance is induced;
(iii) activity that promotes the protein expression of the insulin sensitivity factor pAMPKα and inhibits the protein expression of the insulin resistance factor pIRS (Ser302) in adipocytes where insulin resistance is induced;
(iv) activity that inhibits the gene expression of the insulin resistance factor mTOR or P70S6K and promotes the gene expression of the insulin sensitivity factor AKT, SIRT1, or AMPKα in hepatocytes where insulin resistance is induced; or
(v) activity that inhibits the protein expression of insulin resistance factor pmTOR or pJNK and promotes the protein expression of insulin sensitivity factor pAMPKα, GLUT4 or SIRT1 in hepatocytes where insulin resistance is induced.

5. A composition for promoting muscle formation, anti-obesity, anti-fatty liver, or anti-diabetes, comprising a peptide of any one of claims 1 to 4 as an active ingredient.

6. A pharmaceutical composition for the prevention or treatment of muscle diseases comprising the peptide of claim 1 or claim 2 as an active ingredient.

7. The pharmaceutical composition for the prevention or treatment of muscle diseases of claim 6, wherein the muscle diseases are one or more selected from the group consisting of muscular atrophy, senile sarcopenia, muscular dystrophy, disuse atrophy, spinal muscular amyotrophy, myotonia, muscular hypotonia, muscle weakness, muscle endurance weakness, amyotrophic lateral sclerosis, spinobulbar muscular atrophy, myasthenia gravis, myasthenia, muscle degeneration, and cachexia.

8. A food composition for the prevention or amelioration of muscle diseases comprising the peptide of claim 1 or claim 2 as an active ingredient.

9. The food composition for the prevention or amelioration of muscle diseases of claim 8, wherein the muscle diseases are one or more selected from the group consisting of muscular atrophy, senile sarcopenia, muscular dystrophy, disuse atrophy, spinal muscular amyotrophy, myotonia, muscular hypotonia, muscle weakness, muscle endurance weakness, amyotrophic lateral sclerosis, spinobulbar muscular atrophy, myasthenia gravis, myasthenia, muscle degeneration, and cachexia.

10. A pharmaceutical composition for the prevention or treatment of obesity or fatty liver comprising the peptide of claim 1 or claim 3 as an active ingredient.

11. A food composition for the prevention or amelioration of obesity or fatty liver comprising the peptide of claim 1 or claim 3 as an active ingredient.

12. A pharmaceutical composition for the prevention or treatment of diabetes comprising the peptide of claim 1 or claim 4 as an active ingredient.

13. A food composition for the prevention or amelioration of diabetes comprising the peptide of claim 1 or claim 4 as an active ingredient.
